# EUROPÄISCHE PATENTSCHRIFT

(11) **EP 2 895 470 B1**
(45) Veröffentlichungstag und Bekanntmachung des Hinweises auf die Patenterteilung: **12.02.2020**
(21) Anmeldenummer: 12758867.1
(22) Anmeldetag: 14.09.2012
(51) Int. Cl.: C07D 313/00

(54) **UNGESÄTTIGTE LACTONE ALS RIECHSTOFFE**
UNSATURATED LACTONES AS FRAGRANTS
LACTONES INSATUREES COMME INGREDIENTS PARFUMANTS

(43) Veröffentlichungstag der Anmeldung: 22.07.2015
(73) Patentinhaber: Symrise AG, 37603 Holzminden (DE)
(72) Erfinder: HÖLSCHER, Bernd, 37620 Halle (DE); MANSFELD, Marc, 37647 Brevörde (DE); WAGNER, Tobias, 37627 Hellental (DE)
(74) Vertreter: Fabry, Bernd
(86) Internationale Anmeldenummer: PCT/EP2012/068108
(87) Internationale Veröffentlichungsnummer: WO 2014/040636

(56) Entgegenhaltungen:
- PETASIS, N. A. ET AL.: "Alicyclic Claisen rearrangement with a two-atom ring contraction. A new synthetic approach to cembranoids", TETRAHEDRON LETTERS, Bd. 34, Nr. 11, 12. März 1993 (1993-03-12) , Seiten 1721-1724, XP002696581, ELSEVIER SCIENCE PUBLISHERS, AMSTERDAM; NL DOI: 10.1016/S0040-4039(00)60761-X

## Beschreibung

Die vorliegende Erfindung betrifft Verbindungen der Formel (Ia) oder (Ib), Mischungen umfassend oder bestehend aus Verbindungen der Formel (Ia) oder (Ib) sowie parfümierte Erzeugnisse umfassend eine (vorzugsweise sensorisch wirksame) Menge einer Verbindung der Formel (Ia) oder (Ib) bzw. einer erfindungsgemäßen Mischung.

Zusätzlich betrifft die vorliegende Erfindung die Verwendung der erfindungsgemäßen Verbindungen, erfindungsgemäßen Mischungen bzw. erfindungsgemäßen Erzeugnisse als Riechstoff bzw. Riechstoffmischung bzw. -erzeugnis.

In einem weiteren Aspekt betrifft die vorliegende Erfindung ein Verfahren zum Vermitteln eines Geruches und ein Verfahren zur Herstellung erfindungsgemäßer Verbindungen.

Weitere Aspekte der Erfindung und besonders bevorzugte Ausgestaltungen ergeben sich aus der nachfolgenden Beschreibung und den beigefügten Patentansprüchen.

Trotz einer Vielzahl bereits vorhandener Riechstoffe besteht in der Parfümindustrie weiterhin ein genereller Bedarf an Riechstoffen, die einerseits neue Geruchsnoten vermitteln oder andererseits durch neue Verbindungen eine bereits bekannte Geruchsnote vermitteln. So besteht regelmäßig Bedarf an Verbindungen, die die Geruchsnote Moschus vermitteln. Von besonderem Interesse sind Verbindungen (Riechstoffe), die in der Lage sind, in Erzeugnissen neben der Geruchsnote Moschus weitere angenehme Geruchsnoten zu vermitteln. Besonders wünschenswert sind Überlagerungen unterschiedlicher geruchlicher Aspekte und Geruchsnoten, so dass dadurch ein insgesamt komplexer Geruchseindruck entsteht. Insbesondere besteht ein Interesse an Riechstoffen mit Moschus-Geruchsnoten, welche in der Lage sind, eine harmonische Kombination mit blumig duftenden Riechstoffen einzugehen und dabei einen angenehmen komplexen Geruchseindruck erzeugen. Solche Verbindungen mit ihren neuartigen bzw. originellen Geruchsnoten bzw. komplexen Geruchseindrücken erweitern die Möglichkeiten des Parfümeurs. Geeignete Riechstoffe sollten neben einer Moschus-Geruchsnote vorzugsweise weitere Geruchsnoten und Aspekte aufweisen, und dadurch einer modernen Duftkomposition geruchlichen Charakter und Komplexität verleihen.

Neben geeigneten geruchlichen Eigenschaften einer Verbindung als Riechstoff sind zusätzliche Sekundäreigenschaften von großer Bedeutung. Dazu zählen Eigenschaften wie Stabilität, Kompatibilität mit anderen Verbindungen in einer Duftkomposition, Ausgiebigkeit, Löslichkeit, Bioabbaubarkeit sowie toxikologische Unbedenklichkeit. Weitere interessante Eigenschaften betreffen das Haftvermögen, die Retention bzw. die Substantivität eines Riechstoffes, insbesondere auf Fasern und/oder Haaren. Von besonderer Bedeutung ist diese Eigenschaft für tensidhaltige Produkte wie Shampoos, Waschmittel und Weichspüler.

Als Haftvermögen bezeichnet man die intrinsische Fähigkeit einer Verbindung, auf einem Substrat zu haften. Als Substantivität bzw. Retention bezeichnet man die Fähigkeit einer Verbindung, aus einer, meist wässrigen, Phase heraus auf ein Substrat aufzuziehen bzw. - z.B. auch nach einem Wasch- oder Spülvorgang - auf einem Substrat zu verbleiben. Dieser Effekt zeigt sich insbesondere auf Substraten wie Haut, Haar und textilen Fasern (z.B. Wolle, Baumwolle, Leinen, synthetische Fasern). Die Begriffe "Substantivität" und "Retention" werden z.B. in EP 1 201 738 A1 ausführlich erläutert (vergleiche dort die Abschnitte [0004]-[0005]). Gesucht werden generell Riechstoffe mit hoher Substantivität bzw. Retention. PETASIS, N. A. ET AL.: "Alicyclic Claisen rearrangement with a two-atom ring contraction. A new synthetic approach to cembranoids",TETRAHEDRON LETTERS, Bd. 34, Nr. 11, 12. März 1993 (1993-03-12), Seiten 1721-1724, XP002696581,ELSEVIER SCIENCE PUBLISHERS, AMSTERDAM; NL DOI: 10.1016/S0040-4039(00)60761-X beschreibt eine Verbindung ähnlich des vorliegenden Anspruchs. Das unterscheidende Merkmal ist die Methylgruppe am 14. Kohlenstoffatom.

Der Prozess, Verbindungen zu identifizieren, die die zuvor beschriebenen, positiven Eigenschaften aufweisen, ist häufig mühselig und nicht vorhersehbar, da
- die Mechanismen der Geruchswahrnehmung nicht ausreichend bekannt sind,
- die Zusammenhänge zwischen der spezifischen Geruchswahrnehmung (Geruchsidentifikation) einerseits und der chemischen Struktur des entsprechend zugehörigen Riechstoffes andererseits nicht hinreichend erforscht sind, und
- bereits geringfügige Änderungen am strukturellen Aufbau eines bekannten Riechstoffs häufig starke Änderungen der sensorischen Eigenschaften bewirken und häufig die Verträglichkeit für den menschlichen Organismus beeinträchtigen (d.h. die Verträglichkeit reduzieren).

Bekannte Verbindungen, die die Geruchsnote Moschus vermitteln, wurden in eigenen, früheren Untersuchungen und Ergebnissen wie folgt beschrieben:
EP 0 908 455 B1 betrifft eine Riechstoffkomposition enthaltend 13-Methyloxacyclo-pentadec-10-en-2-on. Eigene Untersuchungen haben gezeigt, dass diese Verbindungen regelmäßig eine pudrige, moschusartige, lactonartige, fruchtige Geruchsnote vermitteln.
EP 0 424 787 B1 betrifft ein Gemisch, enthaltend mindestens 60 Gew.%, bezogen auf das Gewicht des Gemisches, Pentadecenolide und weitere Bestandteile. Eigene Untersuchungen haben gezeigt, dass dieses Gemisch regelmäßig eine moschusartigambrierte, fruchtige, birnenartige Geruchsnote vermittelt.

Es war die primäre Aufgabe der vorliegenden Erfindung, neue Verbindungen als Riechstoffe zu identifizieren, die die Geruchsnote Moschus vermitteln, vorzugsweise einhergehend mit zusätzlichen interessanten und originellen geruchlichen Eigenschaften. Weiterhin sollen diese Verbindungen neue und originelle parfümierte Erzeugnisse mit besonderen geruchlichen Noten und Aspekten bzw. mit besonderer geruchlicher Komplexität ermöglichen. Insbesondere war es Aufgabe der vorliegenden Erfindung, Riechstoffe zu identifizieren, die die Geruchsnote Moschus vermitteln und zudem in positiver Weise ein, zwei oder sämtliche der oben genannten und/oder der nachstehend angegebenen bevorzugten Sekundäreigenschaften aufweisen und weiterhin zur Kombination mit Riechstoffen geeignet sind, welche eine blumige und/oder grün-fruchtige (eine grün-fruchtige Geruchsnote wird fehlerfrei sowohl als grüne Geruchsnote als auch als fruchtige Geruchsnote wahrgenommen) und/oder holzige Geruchsnote vermitteln. Bevorzugte Sekundäreigenschaften sind insbesondere (i) höhere Stabilität unter bestimmten Anwendungsbedingungen, (ii) eine höhere Ausgiebigkeit, (iii) ein besseres Haftungsvermögen, (iv) eine hohe Substantivität, (v) eine bemerkenswerte Booster-Wirkung oder ein starkes Blooming (vi), bessere dermatologische und toxikologische Eigenschaften gegenüber vergleichbaren Riechstoffen und (vii) eine hohe Retention.

Diese Aufgabe wird erfindungsgemäß gelöst durch eine
Verbindung der Formel (Ia) oder (Ib) 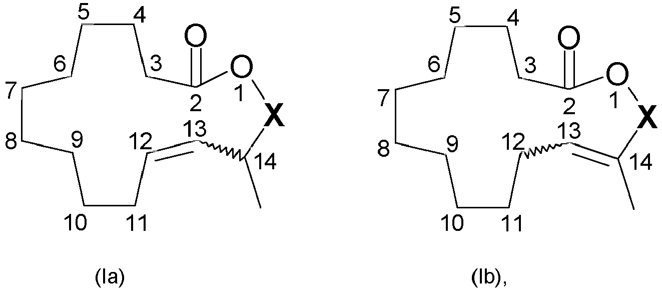 wobei unabhängig voneinander
in jeder der Formeln (Ia) und (Ib)
X eine Methylen- oder Ethylen-Gruppe bedeutet,
und
die gewellte Linie bedeutet, dass die Konfiguration an der zugeordneten Doppelbindung E oder Z ist
und
wobei in der Formel (Ia) die Konfiguration am Kohlenstoffatom mit der Nummer 14 R oder S ist.

Bei den erfindungsgemäßen Verbindungen der Formel (Ia) bzw. (Ib) handelt es sich um bestimmte cyclische, ungesättigte Lactone.

In eigenen Untersuchungen hat sich überraschenderweise gezeigt, dass die erfindungsgemäßen Verbindungen regelmäßig eine, zwei, mehrere oder sämtliche der Geruchsnoten Moschus, strahlend, weich, holzig, erogen, blumig und exaltierend, insbesondere Moschus, vermitteln und insbesondere zur Kombination mit Riechstoffen geeignet sind, welche eine blumige und/oder holzige und/oder grün-fruchtige Geruchsnote vermitteln. Zusätzlich zu den gewünschten (und zuvor genannten) geruchlichen Eigenschaften, weisen die erfindungsgemäßen Verbindungen der Formel (Ia) bzw. (Ib) insbesondere ein, zwei oder sämtliche der oben genannten Sekundäreigenschaften auf, regelmäßig in verglichen mit Verbindungen, die sonst sehr ähnliche oder identische geruchliche Eigenschaften aufweisen, überragender Weise.

Die Ergebnisse sind insofern überraschend, als dass markocyclische Moschusverbindungen allgemein als gut untersucht galten und die erfindungsgemäßen ungesättigten Lactone der Formel (Ia) bzw. (Ib) neue, wertvolle Riechstoffe mit der Geruchsnote Moschus darstellen. Die erfindungsgemäßen Verbindungen der Formel (Ia) bzw. (Ib) verfügen über ganz eigenständige olfaktorische Eigenschaften, die sich deutlich von den bekannten Riechstoffen abheben und diese zum Teil auch übertreffen. Dass diese erfindungsgemäßen Verbindungen die oben beschriebenen gewünschten Eigenschaften aufweisen, war nicht zu erwarten und besonders überraschend.

Die Geruchseigenschaften der erfindungsgemäßen Verbindungen wurden in eigenen Untersuchungen von geschulten Paneltestern als moschusartig, strahlend, weich, holzig, erogen, blumig und/oder exaltierend beschrieben. Die erfindungsgemäßen Verbindungen erinnern an die Geruchsnote Moschuskörneröl und vermitteln einen sehr natürlichen Geruchseindruck

Die erfindungsgemäßen Verbindungen der Formel (Ia) besitzen ein Chiralitätszentrum am Kohlenstoffatom 14. Daher liegen diese Verbindungen entweder S- oder R-konfiguriert vor.

Wie oben beschrieben bedeutet die gewellte Linie, dass die Konfiguration an der zugeordneten Doppelbindung E oder Z ist. Daher liegen die Verbindungen der Formel (Ia) (die sowohl S- als auch R-konfiguriert vorliegen können) bzw. (Ib), als E- oder Z-konfiguriert vor.

Bevorzugt sind erfindungsgemäße Verbindungen der Formel (Ia) bzw. (Ib) (wie oben beschrieben), die aus der folgenden Gruppe ausgewählt sind:

| **Formel** | **X** | **E/Z** | **R/S** | **Strukturformel** | **IUPAC Name** |
|---|---|---|---|---|---|
| (Ia-1) | CH₂ | E | 14R | 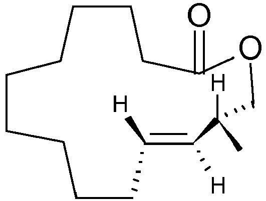 | (12E,14R)-14-methyl-1-oxacyclopentadec-12-en-2-one |
| (Ia-2) | CH₂ | E | 14S | 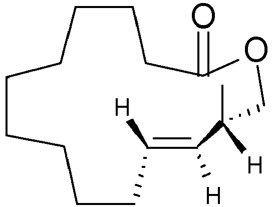 | (12E,14S)-14-methyl-1-oxacyclopentadec-12-en-2-one |
| (Ia-3) | C₂H₄ | E | 14S | 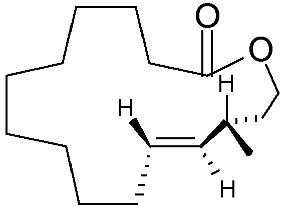 | (12E,14S)-14-methyl-1-oxacyclohexadec-12-en-2-one |
| (Ia-4) | C₂H₄ | E | 14R | 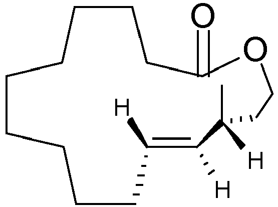 | (12E,14R)-14-methyl-1-oxacyclohexadec-12-en-2-one |
| (Ia-5) | CH₂ | Z | 14S | 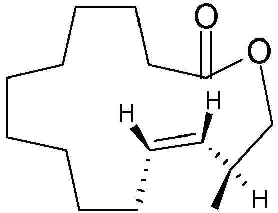 | (12Z,14S)-14-methyl-1-oxacyclopentadec-12-en-2-one |
| (Ia-6) | CH₂ | Z | 14R | 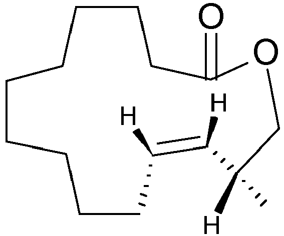 | (12Z,14R)-14-methyl-1-oxacyclopentadec-12-en-2-one |
| (Ia-7) | C₂H₄ | Z | 14R | 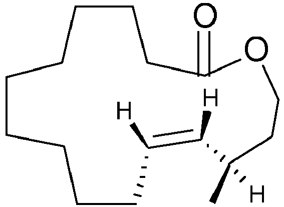 | (12Z,14R)-14-methyl-1-oxacyclohexadec-12-en-2-one |
| (Ia-8) | C₂H₄ | Z | 14S | 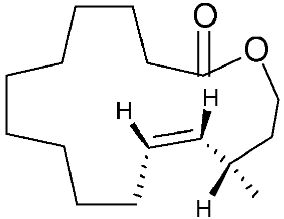 | (12Z,14S)-14-methyl-1-oxacyclohexadec-12-en-2-one |
| (Ib-1) | CH₂ | Z | --- | 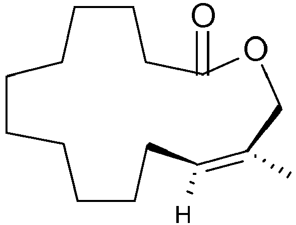 | (13Z)-14-methyl-1-oxacyclopentadec-13-en-2-one |
| (Ib-2) | CH₂ | E | --- | 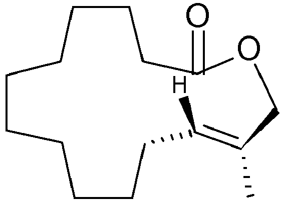 | (13E)-14-methyl-1-oxacyclopentadec-13-en-2-one |
| (Ib-3) | C₂H₄ | Z | --- | 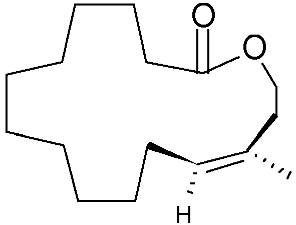 | (13Z)-14-methyl-1-oxacyclohexadec-13-en-2-one |
| (Ib-4) | C₂H₄ | E | --- | 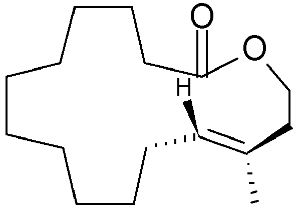 | (13E)-14-methyl-1-oxacyclohexadec-13-en-2-one |

Gemäß einer bevorzugten Anwendung sind Verbindungen der Formel (Ia) bzw. (Ib) bevorzugt, wobei X sowohl für sämtliche der Verbindungen der Formel (Ia) als auch für sämtliche der Verbindungen der Formel (Ib) eine Methylengruppe (X = CH₂) bedeutet. Gemäß einer weiteren bevorzugten Anwendung sind Verbindungen der Formel (Ia) bzw. (Ib) bevorzugt, wobei X sowohl für sämtliche der Verbindungen der Formel (Ia) als auch für sämtliche der Verbindungen der Formel (Ib) eine Ethylengruppe (X = C₂H₄) bedeutet.

Verbindungen der Formel (Ia), wobei X eine Methylengruppe (X = CH₂) bedeutet, sind besonders bevorzugt, da diese in eigenen Untersuchungen die gewünschte Geruchsnote in besonders vorteilhafter Weise vermitteln und zusätzlich die gewünschten (oben beschriebenen) Sekundäreigenschaften aufweisen.

Je nach gewünschter Anwendung können auch Verbindungen der Formel (Ib) bevorzugt sein.

Die vorliegende Erfindung betrifft auch eine Mischung umfassend oder bestehend aus zwei oder mehr erfindungsgemäßen Verbindungen (wie oben beschrieben). Dabei gilt für die bevorzugt enthaltenen Verbindungen der Formel (Ia) bzw. (Ib) das oben Gesagte entsprechend.

Grundsätzlich bevorzugt sind erfindungsgemäße Mischungen (wie oben beschrieben)
- umfassend eine Verbindung oder mehrere Verbindungen der Formel (Ia) und eine Verbindung oder mehrere Verbindungen der Formel (Ib), wobei X für sämtliche der Verbindungen der Formel (Ia) und (Ib) die gleiche Bedeutung hat und eine Methylen- oder Ethylen-Gruppe bedeutet
   und/oder
- umfassend zwei oder mehr Verbindungen der Formel (Ia), wobei X für beide oder sämtliche dieser Verbindungen die gleiche Bedeutung hat und eine Methylengruppe bedeutet
   und/oder
- umfassend ein E-Isomer sowie ein Z-Isomer einer Verbindung der Formel (Ia), wobei X sowohl für das E-Isomer als auch für das Z-Isomer die gleiche Bedeutung hat und eine Methylen- oder Ethylen-Gruppe bedeutet
   und/oder
- umfassend beide Enantiomere eines Enantiomerenpaares von Verbindungen der Formel (Ia) wie oben definiert, vorzugsweise ein Racemat zweier Enantiomere von Verbindungen der Formel (Ia) wie oben definiert
   und/oder
- umfassend ein E-Isomer und ein Z-Isomer einer Verbindung der Formel (Ib), wobei X sowohl für das E-Isomer als auch für das Z-Isomer die gleiche Bedeutung hat und eine Methylen- oder Ethylen-Gruppe bedeutet.

In einer bevorzugten Anwendung umfasst eine erfindungsgemäße Mischung (wie oben beschrieben) eine Verbindung oder mehrere Verbindungen der Formel (Ia) und eine Verbindung oder mehrere Verbindungen der Formel (Ib), wobei X für sämtliche der Verbindungen der Formel (Ia) und (Ib) die gleiche Bedeutung hat und eine Methylen-gruppe bedeutet. Vorzugsweise umfasst eine solche erfindungsgemäße Mischung sämtliche Verbindungen der Formel (Ia), bei denen X eine Methylengruppe bedeutet, und sämtliche der Verbindungen der Formel (Ib), bei denen X eine Methylengruppe bedeutet, d.h. eine solche bevorzugte erfindungsgemäße Mischung umfasst:
Verbindung der Formel (Ia-1) und
Verbindung der Formel (Ia-2) und
Verbindung der Formel (Ia-5) und
Verbindung der Formel (Ia-6) und
Verbindung der Formel (Ib-1) und
Verbindung der Formel (Ib-2).

Bevorzugte erfindungsgemäße Mischungen (wie oben beschrieben) umfassen zwei oder mehr Verbindungen der Formel (Ia), wobei X für beide oder sämtliche dieser Verbindungen die gleiche Bedeutung hat und eine Methylengruppe bedeutet, und/oder ein E-Isomer sowie ein Z-Isomer einer Verbindung der Formel (Ia), wobei X sowohl für das E-Isomer als auch für das Z-Isomer die gleiche Bedeutung hat und eine Methylengruppe bedeutet.

In einer besonders bevorzugten Ausführungsform umfasst eine erfindungsgemäße Mischung (wie oben beschrieben, vorzugsweise wie oben als bevorzugt definiert) ein E-Isomer sowie ein Z-Isomer einer Verbindung der Formel (Ia), wobei X sowohl für das E-Isomer als auch für das Z-Isomer die gleiche Bedeutung hat und eine Methylen-Gruppe bedeutet.

Bei den folgenden Mischungen handelt es sich um beispielhaft aufgeführte, bevorzugte Kombinationen von Verbindungen der Formel (Ia), die für die Zwecke der vorliegenden Erfindung (wie hierin beschrieben) besonders geeignet und dementsprechend bevorzugt sind:
Verbindung der Formel (Ia-1) und Verbindung der Formel (Ia-5), oder
Verbindung der Formel (Ia-1) und Verbindung der Formel (Ia-6), oder
Verbindung der Formel (Ia-2) und Verbindung der Formel (Ia-5), oder
Verbindung der Formel (Ia-2) und Verbindung der Formel (Ia-6).

In einer weiteren, ebenfalls bevorzugten Ausführungsform umfasst eine erfindungsgemäße Mischung (wie oben beschrieben, vorzugsweise wie oben als bevorzugt definiert) (i) zwei E-Isomere einer Verbindung der Formel (Ia) und/oder (ii) zwei Z-Isomere einer Verbindung der Formel (Ia), wobei X sowohl für die zwei E-Isomere als auch für die zwei Z-Isomere die gleiche Bedeutung hat und eine Methylen-Gruppe bedeutet.

Bei den folgenden Mischungen handelt es sich um weitere beispielhaft aufgeführte, bevorzugte Kombinationen von Verbindungen der Formel (Ia), die für die Zwecke der vorliegenden Erfindung (wie hierin beschrieben) besonders geeignet und dementsprechend bevorzugt sind:
Verbindung der Formel (Ia-1) und Verbindung der Formel (Ia-2), oder
Verbindung der Formel (Ia-5) und Verbindung der Formel (Ia-6), oder
Verbindung der Formel (Ia-1), Verbindung der Formel (Ia-2), Verbindung der Formel (Ia-5) und Verbindung der Formel (Ia-6).

In einer weiteren, ebenfalls bevorzugten Ausführungsform umfasst eine erfindungsgemäße Mischung (wie oben beschrieben, vorzugsweise wie oben als bevorzugt definiert)
(i) ein E-Isomer einer Verbindung der Formel (Ia) und zwei Z-Isomere einer Verbindung der Formel (Ia), wobei X sowohl für das eine E-Isomer als auch für die zwei Z-Isomere die gleiche Bedeutung hat und eine Methylen-Gruppe bedeutet,
   oder
(ii) zwei E-Isomere einer Verbindung der Formel (Ia) und ein Z-Isomer einer Verbindung der Formel (Ia), wobei X sowohl für die zwei E-Isomere als auch für das eine Z-Isomer die gleiche Bedeutung hat und eine Methylen-Gruppe bedeutet.

Bei den folgenden Mischungen handelt es sich um weitere beispielhaft aufgeführte, bevorzugte Kombinationen von Verbindungen der Formel (Ia), die für die Zwecke der vorliegenden Erfindung (wie hierin beschrieben) besonders geeignet und dementsprechend bevorzugt sind:
Verbindung der Formel (Ia-1), Verbindung der Formel (Ia-2) und Verbindung der Formel (Ia-5), oder
Verbindung der Formel (Ia-1), Verbindung der Formel (Ia-2) und Verbindung der Formel (la-6), oder
Verbindung der Formel (Ia-5), Verbindung der Formel (Ia-6) und Verbindung der Formel (Ia-1), oder
Verbindung der Formel (Ia-5), Verbindung der Formel (Ia-6) und Verbindung der Formel (Ia-2).

In einer nächsten bevorzugten Ausführungsform umfasst eine erfindungsgemäße Mischung (wie oben beschrieben, vorzugsweise wie oben als bevorzugt definiert) ein Racemat von Verbindungen der Formel (Ia).

Selbstverständlich gilt im Zusammenhang mit den vorstehend (als auch nachstehend) beschriebenen Mischungen auch, dass diese nicht nur die oben beschriebenen erfindungsgemäßen Verbindungen umfassen bzw. enthalten können, sondern auch allein aus Kombinationen dieser Verbindungen (insbesondere solchen wie vorstehend beschrieben) bestehen können.

Je nach gewünschter Anwendung können auch erfindungsgemäße Mischungen (wie oben beschrieben, vorzugsweise wie oben als bevorzugt definiert) bevorzugt sein, die Verbindungen der Formel (Ib) umfassen oder daraus bestehen. Selbstverständlich können solche Mischungen zudem eine oder mehrere Verbindungen der Formel (Ia) enthalten.

In einer anderen bevorzugten Anwendung umfasst eine erfindungsgemäße Mischung (wie oben beschrieben) eine Verbindung oder mehrere Verbindungen der Formel (Ia) und eine Verbindung oder mehrere Verbindungen der Formel (Ib), wobei X für sämtliche der Verbindungen der Formel (Ia) und (Ib) die gleiche Bedeutung hat und eine Ethylengruppe bedeutet. Vorzugsweise umfasst eine solche erfindungsgemäße Mischung sämtliche Verbindungen der Formel (Ia), bei denen X eine Ethylengruppe bedeutet, und sämtliche der Verbindungen der Formel (Ib), bei denen X eine Ethylengruppe bedeutet, d.h. eine solche bevorzugte erfindungsgemäße Mischung umfasst:
Verbindung der Formel (Ia-3) und
Verbindung der Formel (Ia-4) und
Verbindung der Formel (Ia-7) und
Verbindung der Formel (Ia-8) und
Verbindung der Formel (Ib-3) und
Verbindung der Formel (Ib-4).

Ein weiterer Aspekt der vorliegenden Erfindung betrifft ein Erzeugnis bestehend aus oder umfassend
(a) eine, zwei oder mehr erfindungsgemäße Verbindungen, vorzugsweise zumindest eine, zwei oder mehr Verbindungen der Formel (Ia), wobei X für die eine, beide oder sämtliche dieser Verbindungen die gleiche Bedeutung hat und eine Methylengruppe bedeutet
   und/oder
   ein E-Isomer sowie ein Z-Isomer einer Verbindung der Formel (Ia), wobei X sowohl für das E-Isomer als auch für das Z-Isomer die gleiche Bedeutung hat und eine Methylen- oder Ethylen-Gruppe bedeutet
   und/oder
   beide Enantiomere eines Enantiomerenpaares von erfindungsgemäßen Verbindungen der Formel (Ia) (wie oben beschrieben, vorzugsweise wie oben als bevorzugt definiert), vorzugsweise ein Racemat zweier Enantiomere von erfindungsgemäßen Verbindungen (wie oben beschrieben, vorzugsweise wie oben als bevorzugt definiert) der Formel (Ia),
   und/oder
   ein E-Isomer und ein Z-Isomer einer Verbindung der Formel (Ib), wobei X sowohl für das E-Isomer als auch für das Z-Isomer die gleiche Bedeutung hat und eine Methylen- oder Ethylen-Gruppe bedeutet
   sowie
   ein, zwei oder mehr weitere Bestandteile.

Es hat sich in eigenen Untersuchungen gezeigt, dass solche, die Geruchsnote Moschus vermittelnden, erfindungsgemäßen Erzeugnisse (zu den Erzeugnissen im Einzelnen siehe weiter unten im Text) regelmäßig eine angenehme Ausstrahlung und/oder Harmonie aufweisen und/oder eine vorhandene Geruchsnote verstärken.

Ein bevorzugtes erfindungsgemäßes Erzeugnis umfasst als Komponente
(b) eine, zwei oder mehrere Verbindungen, die keine erfindungsgemäßen Verbindungen (wie oben beschrieben) sind, wobei diese Verbindung bzw. diese Verbindungen ausgewählt sind aus der Gruppe bestehend aus Riechstoffen
wobei
(I) die Gesamtmenge an erfindungsgemäßen Verbindungen (wie oben beschrieben, vorzugsweise wie oben als bevorzugt definiert) in dem Erzeugnis ausreicht, eine oder mehrere der Geruchsnoten moschusartig, strahlend, weich, holzig, erogen, blumig und exaltierend zu vermitteln,
   und/oder
(II) die Gesamtmenge an Verbindungen der Komponente (b) in dem Erzeugnis ausreicht, eine, mehrere oder sämtliche der Noten holzig, blumig, frisch, grün, fruchtig, moschusartig und würzig-balsamisch zu vermitteln, vorzugsweise eine oder sämtliche der Noten holzig und blumig,
   wobei vorzugsweise die Gesamtmenge an erfindungsgemäßen Verbindungen (wie oben beschrieben, vorzugsweise wie oben als bevorzugt definiert) in dem Erzeugnis ausreicht, eine oder mehrere dieser Noten zu verstärken und/oder zu modifizieren,
   und/oder
(III) die Gesamtmenge an erfindungsgemäßen Verbindungen (wie oben beschrieben, vorzugsweise wie oben als bevorzugt definiert) in dem Erzeugnis ausreicht, dem Erzeugnis einen Eindruck von Frische, Sauberkeit, Harmonie, Ausstrahlung und/oder Natürlichkeit zu verleihen,
   und/oder
(IV) die Gesamtmenge an erfindungsgemäßen Verbindungen (wie oben beschrieben, vorzugsweise wie oben als bevorzugt definiert) in dem Erzeugnis ausreicht, um im Vergleich zu einem Vergleichserzeugnis, das bei ansonsten identischer Zusammensetzung keine erfindungsgemäßen Verbindungen (wie oben beschrieben) umfasst, einen intensiveren, harmonischeren, hochwertigeren, helleren, weicheren, abgerundeteren und/oder natürlicheren Geruchseindruck zu bewirken.

Durch Kombination erfindungsgemäßer Verbindungen der Formel (Ia) bzw. (Ib) mit ein, zwei oder mehreren Riechstoffen gemäß Komponente (b) lassen sich neue Riechstoffkompositionen zusammenstellen. Auf diese Weise lassen sich besonders interessante, neue und originelle Geruchsnoten kreieren bzw. kombinieren. Besonders bevorzugt ist dabei die Kombination mit Riechstoffen, die keine erfindungsgemäßen Verbindungen (wie nachfolgend beschrieben) sind und eine holzige und/oder blumige und/oder grün-fruchtige Geruchsnote vermitteln, besonders bevorzugt eine blumige und/oder grün-fruchtige Geruchsnote.

Riechstoffe (Komponente (b)), die zur Kombination mit erfindungsgemäßen Verbindungen besonders geeignet sind, werden z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001 genannt. Im Einzelnen seien folgende Verbindungen genannt:
- Riechstoffe, die in Extrakten aus natürlichen Rohstoffen enthalten sind, wie zum Beispiel in ätherischen Ölen, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-ÖI; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litseacubeba-Öl; Lorbeerblätteröl; Macisöl; Majoranöl; Mandarinenöl; Massoirindenöl; Mimosa-Absolue; Moschuskörneröl; Mo-schustinktur; Muskateller-SalbeiÖl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie in Fraktionen davon, insbesondere daraus isolierte Riechstoffe;
- Einzel-Riechstoffe aus der Gruppe der Kohlenwasserstoffe, wie z.B. 3-Caren; a-Pinen; b-Pinen; a-Terpinen; g-Terpinen; p-Cymol; Bisabolen; Camphen; Caryo-phyllen; Cedren; Farnesen; Limonen; Longifolen; Myrcen; Ocimen; Valencen; (E,Z)-1,3,5-Undecatrien; Styrol; Diphenylmethan;
- aliphatische Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
- aliphatische Aldehyde und deren Acetale wie z.B. Hexanal; Heptanal; Octa-nal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
- aliphatische Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
- aliphatische schwefelhaltige Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
- aliphatische Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
- Ester aliphatischer Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, Isoamylbutyrat; Hexylbutyrat; (E)- und (Z)-3-Hexenyl-isobutyrat; Hexylcrotonat; Ethylisovaleria-nat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
- acyclische Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
- acyclische Terpenaldehyde und -ketone wie z. B. Citronellal;; 7-Methoxy-3,7-dimethyloctanal; 2,6,10-Trimethyl-9-undecenal; Geranylaceton; sowie die Dimethyl- und Diethylacetale von Geranial, Neral,
- cyclische Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoa-te, Crotonate, Tiglinate und 3-Methyl-2-butenoate;
- cyclische Terpenaldehyde und -ketone wie z. B. Menthon; Isomenthon ; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
- cyclische Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
- cycloaliphatische Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
- cyclische und cycloaliphatische Ether wie z.B. Cineol; Cedrylmethylether; Cyclododecylmethylether;1,1-Dimethoxycyclododecan; (Ethoxymethoxy)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1-b]furan; 1,5,9-Tri-methyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
- cyclische und makrocyclische Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
- cycloaliphatische Aldehyde wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- cycloaliphatische Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
- Ester cyclischer Alkohole wie z.B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat;2-Cyclo-pentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat;
- Ester cycloaliphatischer Alkohole wie z.B.1-Cyclohexylethylcrotonat;
- Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
- araliphatische Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
- Ester araliphatischer Alkohole und aliphatischer Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenyl-ethylisovalerianat; 1-Phenylethylacetat; alpha-Trichlormethylbenzylacetat; alpha ,alpha-Dimethylphenylethylacetat; alpha,alpha-Dimethylphenylethylbutyrat; Cinnamylacetat; 2-Phenoxyethylisobutyrat; 4-Methoxybenzylacetat;
- araliphatische Ether wie z.B. 2-Phenylethylmethylether; 2-Phenylethyl-isoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
- aromatische und araliphatische Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butyl-phenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
- aromatische und araliphatische Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-aceto-naphthon;
- aromatische und araliphatische Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
- stickstoffhaltige aromatische Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiffsche Basen von Methylanthranilat mit 7-Hydroxy-3,7-dimethyloctanal, 2-Methyl-3-(4-tert.-butyl-phenyl)propanal oder 2,4-Dimethyl-3-cyclohexencarbaldehyd; 6-Isopropyl-chinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
- Phenole, Phenylether und Phenylester wie z.B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthyliso-butylether; 1,4-Dimethoxybenzol; Eugenylacetat; 2-Methoxy-4-methylphenol; 2-Ethoxy-5-(1-propenyl)phenol; p-Kresylphenylacetat;
- heterocyclische Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 2,3-Dihydrocumarin; Octahydrocumarin.

Bevorzugte erfindungsgemäße Erzeugnisse (wie oben beschrieben) umfassen eine Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine holzige Geruchsnote vermitteln.

Besonders zur Kombination geeignete holzige Riechstoffe sind: Sandranol (2-Ethyl-4-(2,2,3)-trimethylcyclopent-3-yl-but-2-en-1-ol), Ysamber K (1',1',5',5'-Tetramethylhexahydro-spiro[1,3-dioxolan-2,8'(5'H)-2H-2,4a-methanonaphthalen]), Timberol (1-(2,2,6-Trimethylcyclohexyl)hexan-3-ol), Iso-E-Super (2,3,8,8,-Tetramethyl-1,2,3,4,5,6,8-octahydro-2-naphthalenyl-methylketon), Isobornylacetat (2-exo-Bornanylacetat), Ylanat (2-tert-Butylcyclohexylacetat).

Diese Erzeugnisse, umfassend die Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine holzige Geruchsnote vermitteln, wurden in eigenen Untersuchungen häufig mit einem helleren, natürlicheren, frischeren, strahlenderen und sauberen Geruchseindruck bewertet, verglichen mit Vergleichserzeugnissen, die bei sonst identischer Zusammensetzung keine erfindungsgemäßen Verbindungen (wie oben beschrieben) enthielten.

Andere bevorzugte erfindungsgemäße Erzeugnisse (wie oben beschrieben) umfassen eine Kombination von (i) erfindungsgemäßen Verbindungen (wie oben beschrieben, vorzugsweise wie oben als bevorzugt definiert) und (ii) ein, zwei oder mehreren Verbindungen ausgewählt aus der Gruppe bestehend aus Riechstoffen (Komponente (b)), die eine blumige Geruchsnote vermitteln.

Besonders zur Kombination geeignete blumige Riechstoffe sind: Lilial (2-Methyl-3-(4-tert-butylphenyl)propanal), Hedion (Methyl (3-oxo-2-pentylcyclopentyl)acetat), Mayol (4-Isopropyl-cyclohexyl) methanol), Linalool (3,7-Dimethyl-1,6-octadien-3-ol), Dihydromyrcenol (2,6-Dimethyl-7-octen-2-ol), Citronellol (3,7-Dimethyl-6-octen-1-ol), Phenoxanol (3-Methyl-5-Phenyl-pentanol), 2-Phenylethylalkohol, Hydroxycitronellal (3,7 - Dimethyl-7-hydroxyoctan-1-al), alpha-Jonon (4-(2,6,6-Trimethyl-cyclohex-2-enyl)-but-3-en-2-on).

Diese Erzeugnisse, umfassend die Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine blumige Geruchsnote vermitteln, wurden in eigenen Untersuchungen häufig mit einem frischeren, intensiveren, harmonischeren und strahlenderen Geruchseindruck bewertet, verglichen mit Vergleichserzeugnissen, die bei sonst identischer Zusammensetzung keine erfindungsgemäßen Verbindungen (wie oben beschrieben) enthielten. Zusätzlich wurden die blumigen Aspekte regelmäßig als abgerundeter bewertet.

Weitere bevorzugte erfindungsgemäße Erzeugnisse (wie oben beschrieben) umfassen eine Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine grün-fruchtige Geruchsnote vermitteln.

Besonders zur Kombination geeignete grün-fruchtige Riechstoffe sind: Vertral (Octahydro-1H-4,7-methanoinden-5-carbaldehyd), cis-3-Hexen-1-ol, beta-Damascon (1-(2,6,6-Trimethyl-cyclohex-2-enyl)-buten-1-on), Vertocitral (2,4-Dimethylcyclohex-3-en-1-carbaldehyd), Cyclogalbanat (Allyl (Cyclohexyloxy) acetat), Hexylacetat.

Diese Erzeugnisse, umfassend die Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine grün-fruchtige Geruchsnote vermitteln, wurden in eigenen Untersuchungen häufig mit einem runderen und weicheren Geruchseindruck bewertet, verglichen mit Vergleichserzeugnissen, die bei sonst identischer Zusammensetzung keine erfindungsgemäßen Verbindungen (wie oben beschrieben) enthielten. Zudem verleiht die Kombination mit Riechstoffen (Komponente (b)), die eine grün-fruchtige Geruchsnote vermitteln, diesen Erzeugnissen regelmäßig einen gewissen Glanz und vermittelt einen Eindruck von natürlicher(er) Ausstrahlung.

Ebenfalls bevorzugte erfindungsgemäße Erzeugnisse (wie oben beschrieben) umfassen eine Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine würzig-balsamische Geruchsnote vermitteln.

Besonders zur Kombination geeignete würzig-balsamische Riechstoffe sind: Eugenol (2-Methoxy-4-allylphenol), Cumarin (2H-1-Benzopyran-2-on), Anisal-dehyd (4-Methoxybenzaldehyd), Amylzimtaldehyd (2-Penyl-3-phenyl-2-propenal), Isoamylsalicylat (Salicylsäure-3-methylbutylester) und Zimtalkohol (3-Phenyl-2-propen-1-ol).

Diese Erzeugnisse, umfassend die Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine würzig-balsamische Geruchsnote vermitteln, wurden in eigenen Untersuchungen häufig mit einem frischeren, harmonischeren, strahlenderen und natürlicheren Geruchseindruck bewertet, verglichen mit Vergleichserzeugnissen, die bei sonst identischer Zusammensetzung keine erfindungsgemäßen Verbindungen (wie oben beschrieben) enthielten.

Sofern eine Kombination (wie oben beschrieben, vorzugsweise wie oben als bevorzugt definiert) von erfindungsgemäßen Verbindungen (wie oben beschrieben, vorzugsweise wie oben als bevorzugt definiert) und ein, zwei oder mehreren Verbindungen ausgewählt aus der Gruppe bestehend aus Riechstoffen (Komponente (b)), in einem erfindungsgemäßen Erzeugnis (wie oben beschrieben, vorzugsweise wie oben als bevorzugt definiert) vorliegt, werden die erfindungsgemäßen Verbindungen üblicherweise in einer sensorisch wirksamen Menge eingesetzt.

In bevorzugten erfindungsgemäßen Erzeugnissen liegt
die Gesamtmenge an erfindungsgemäßen Verbindungen (wie oben beschrieben, vorzugsweise wie oben als bevorzugt definiert) im Bereich von 0,00001 bis 99,9 Gew.-%, vorzugsweise von 0,001 bis 70 Gew.-% und besonders bevorzugt von 0,01 bis 50 Gew.-% liegt, bezogen auf die Gesamtmenge des Erzeugnisses,
und/oder
das Gewichtsverhältnis der Gesamtmenge an erfindungsgemäßen Verbindungen (wie oben beschrieben, vorzugsweise wie oben als bevorzugt definiert) zu der Gesamtmenge an sonstigen Riechstoffen, die keine erfindungsgemäßen Verbindungen sind, im Bereich von 1:1000 bis 1:0,5, bezogen auf die Gesamtmenge des Erzeugnisses.

Es hat sich in eigenen Untersuchungen gezeigt, dass in erfindungsgemäßen Erzeugnissen (wie oben beschrieben, vorzugsweise wie oben als bevorzugt definiert), die eine wie oben beschriebene Menge erfindungsgemäßer Verbindungen umfassen, sehr häufig die Intensität der Geruchsnoten verstärkt wurde und/oder das Gesamtbild der Geruchsnoten als abgerundeter und/oder harmonischer und/oder natürlicher und/oder strahlender bewertet wurde, verglichen mit Vergleichserzeugnissen, die bei sonst identischer Zusammensetzung keine erfindungsgemäßen Verbindungen (wie oben beschrieben) enthielten. Besonders bevorzugt sind dabei erfindungsgemäße Erzeugnisse, in denen bereits bei Kombination mit sehr geringen Dosierungen erfindungsgemäßer Verbindungen (oder erfindungsgemäßer Mischungen) die oben beschriebenen positiven Effekte zu beobachten waren.

Insbesondere wenn die erfindungsgemäßen Verbindungen dazu eingesetzt werden, eine vorhandene Geruchsnote (vermittelt durch ein, zwei oder mehrere Verbindungen ausgewählt aus der Gruppe bestehend aus Riechstoffen (Komponente (b)) eines Erzeugnisses zu verstärken und/oder abzurunden und/oder harmonischer erscheinen zu lassen und/oder mehr Ausstrahlung zu verleihen und/oder eine spezifische Geruchsnote unter mehreren Geruchsnoten zu betonen, umfassen bevorzugte erfindungsgemäße Erzeugnisse eine geringe Dosierung erfindungsgemäßer Verbindungen bzw. erfindungsgemäßer Mischungen, vorzugsweise eine Gesamtmenge an erfindungsgemäßen Verbindungen im Bereich von 0,01 bis 5 Gew.-%, weiter bevorzugt im Bereich von 0,1 bis 2 Gew.-%, bezogen auf die Gesamtmenge des Erzeugnisses. Besonders bevorzugte sind dabei erfindungsgemäße Erzeugnisse, umfassend die Kombination von (i) erfindungsgemäßen Verbindungen (wie oben beschrieben, vorzugsweise wie oben als bevorzugt definiert) und (ii) einer, zwei oder mehreren Verbindungen ausgewählt aus der Gruppe bestehend aus Riechstoffen (Komponente (b)), die eine holzige, blumige, grün-fruchtige und/oder würzig-balsamische Geruchsnote, vorzugsweise eine holzige und/oder blumige Geruchsnote, vermitteln.

Sofern die erfindungsgemäßen Verbindungen in den oben genannten Gesamtmengen (vorzugsweise in den bevorzugten Gesamtmengen) in einem erfindungsgemäßen Erzeugnis vorliegen (d.h. vorzugsweise in einer entsprechend geringen Dosierung vorliegen), kommt es in Abhängigkeit von den weiteren Komponenten in dem entsprechenden Erzeugnis (zu den weiteren Komponenten siehe weiter unten im Text) in manchen Fällen nicht zu der Vermittlung der Eigengeruchsnote (zu der Eigengeruchsnote siehe weiter oben im Text), vorzugsweise nicht zur Vermittlung der Eigengeruchsnote Moschus.

Bevorzugte erfindungsgemäße Erzeugnisse umfassen daher als Komponente
(b) eine, zwei oder mehrere Verbindungen, die keine erfindungsgemäßen Verbindungen sind, wobei diese Verbindung bzw. diese Verbindungen ausgewählt sind aus der Gruppe bestehend aus Riechstoffen,
mit der Maßgabe, dass
die Verbindung bzw. eine, zwei oder mehrere der Verbindungen eine Moschusriechstoffverbindung ist bzw. Moschusriechstoffverbindungen sind, vorzugsweise eine makrocyclische Moschusriechstoffverbindung ist bzw. makrocyclische Moschusriechstoffverbindungen sind
oder
die Verbindung bzw. eine, zwei oder mehrere der Verbindungen ein Riechstoff bzw. Riechstoffe mit holziger Geruchsnote ist bzw. sind
oder
die Verbindung bzw. eine, zwei oder mehrere der Verbindungen ein Riechstoff bzw. Riechstoffe mit blumiger Geruchsnote ist bzw. sind
oder
die Verbindung bzw. eine, zwei oder mehrere der Verbindungen ein Riechstoff bzw. Riechstoffe mit grün-fruchtiger Geruchsnote ist bzw. sind.

Die zuvor erwähnten nicht erfindungsgemäßen Moschusriechstoffverbindungen umfassen sämtliche Verbindungen, die die Geruchsnote Moschus vermitteln und keine Verbindung der Formel (Ia) oder (Ib) sind. Dabei handelt es sich vorzugsweise um makrocyclische, nicht erfindungsgemäße Moschusriechstoffverbindungen, weiter vorzugsweise makrocyclische, nicht erfindungsgemäße Moschusriechstoffverbindungen ausgewählt aus der Gruppe bestehend aus Nirvanolide, Habanolide und Globalide. Besonders bevorzugt handelt es sich um makrocyclische, nicht erfindungsgemäße Moschusriechstoffverbindungen ausgewählt aus der Gruppe bestehend aus (Z)-13-Methyl-oxacyclopentadec-10-en-2-on, (E,Z)-1-Oxacyclohexadec-12/13-en-2-on und (E,Z)-1-Oxacyclohexadec-12/13-en-2-on.

Zu den Verbindungen von Riechstoffen mit holziger, blumiger oder grün-fruchtiger Geruchsnote siehe weiter oben im Text.

Es hat sich in eigenen Untersuchungen in vielen Fällen besonders deutlich gezeigt, dass erfindungsgemäße Erzeugnisse, umfassend als Komponente (b) eine, zwei oder mehrere Verbindungen, die keine erfindungsgemäßen Verbindungen sind, wobei diese Verbindung bzw. diese Verbindungen ausgewählt sind aus der Gruppe bestehend aus Riechstoffen,
mit der Maßgabe, dass
die Verbindung bzw. eine, zwei oder mehrere der Verbindungen eine Moschusriechstoffverbindung ist bzw. Moschusriechstoffverbindungen sind, vorzugsweise eine makrocyclische Moschusriechstoffverbindung ist bzw. makrocyclische Moschusriechstoffverbindungen sind,
die oben beschrieben positiven Eigenschaften besonders ausgeprägt auftreten. Solche bevorzugten erfindungsgemäßen Erzeugnisse wurden in eigenen Untersuchungen regelmäßig als harmonischer und/oder natürlicher und/oder verstärkter und/oder strahlender und/oder abgerundeter und/oder weicher bewertet, verglichen mit Vergleichserzeugnissen, die bei sonst identischer Zusammensetzung keine erfindungsgemäßen Verbindungen (wie oben beschrieben) enthielten.

Bevorzugte Erzeugnisse, umfassend die Kombination von erfindungsgemäßen Verbindungen (wie oben beschrieben, vorzugsweise wie oben als bevorzugt definiert) und einer, zwei oder mehreren Verbindungen, die keine erfindungsgemäßen Verbindungen sind, wobei diese Verbindung bzw. diese Verbindungen ausgewählt ist bzw. sind aus der Gruppe bestehend aus Riechstoffen,
mit der Maßgabe, dass
die Verbindung bzw. eine, zwei oder mehrere der Verbindungen eine Moschusriechstoffverbindung ist bzw. Moschusriechstoffverbindungen sind, vorzugsweise eine makrocyclische Moschusriechstoffverbindung ist bzw. makrocyclische Moschusriechstoffverbindungen sind
oder
die Verbindung bzw. eine, zwei oder mehrere der Verbindungen ein Riechstoff bzw. Riechstoffe mit holziger Geruchsnote ist bzw. sind
oder
die Verbindung bzw. eine, zwei oder mehrere der Verbindungen ein Riechstoff bzw. Riechstoffe mit blumiger Geruchsnote ist bzw. sind
oder
die Verbindung bzw. eine, zwei oder mehrere der Verbindungen ein Riechstoff bzw. Riechstoffe mit grün-fruchtiger Geruchsnote ist bzw. sind,
haben sich in eigenen Untersuchungen regelmäßig dadurch ausgezeichnet, dass sie ein, zwei oder sämtliche der oben genannten Sekundäreigenschaften in positiver Weise aufweisen, verglichen mit Vergleichserzeugnissen, die bei sonst identischer Zusammensetzung keine erfindungsgemäßen Verbindungen (wie oben beschrieben) enthielten. Dazu gehören insbesondere eine verbesserte Substantivität, Retention und Haftvermögen.

Die erfindungsgemäßen Erzeugnisse (wie oben beschrieben, vorzugsweise wie oben als bevorzugt definiert) enthalten neben erfindungsgemäßen Verbindungen weitere Bestandteile. Solche weiteren Bestandteile können nicht nur Riechstoffe gemäß Komponente (b) (wie oben beschrieben), sondern auch andere/weitere Substanzen bzw. Komponenten sein.

Bevorzugte erfindungsgemäße Erzeugnisse (wie oben beschrieben, vorzugsweise wie oben als bevorzugt definiert), umfassen (zudem)
(c) einen oder mehrere Trägerstoffe für die Komponente (a) und/oder die Komponente (b).

Im Sinne der vorliegenden Erfindung sind Verbindungen der Komponente (a) erfindungsgemäße Verbindungen, wohingegen Verbindungen der Komponente (b) eine, zwei oder mehrere Verbindungen sind, die keine erfindungsgemäßen Verbindungen sind, wobei diese Verbindung bzw. diese Verbindungen ausgewählt sind aus der Gruppe bestehend aus Riechstoffen (sonstige Riechstoffe) (vgl. hierzu die obigen Ausführungen).

Bevorzugte erfindungsgemäße Erzeugnisse sind Erzeugnisse (wie oben beschrieben, vorzugsweise wie oben als bevorzugt definiert), wobei der eine bzw. einer, mehr als einer oder sämtliche der mehreren Trägerstoffe der Komponente (c) ausgewählt ist bzw. sind aus der Gruppe bestehend aus (A) porösen anorganischen Materialien und (B) organischen Materialien, wobei das bzw. die organischen Materialien vorzugsweise Kunststoffe oder sonstige organische Polymere sind oder umfassen.

Poröse anorganische Materialien (A) sind beispielsweise Leichtsulfat, Kieselgele, Zeolithe, Gipse, Tone, Tongranulate, Gasbeton.

Organische Materiealien (B) sind beispielsweise Hölzer, Cellulose-basierende Stoffe, Zucker, Dextrine (z.B. Maltodextrin) oder Kunststoffe wie PVC, Polyvinylacetate oder Polyurethane.

In bevorzugten erfindungsgemäßen Erzeugnissen können die erfindungsgemäßen Verbindungen (wie oben beschrieben, vorzugsweise wie oben als bevorzugt definiert) an einen Trägerstoff (wie oben definiert) adsorbiert vorliegen. Es hat sich in umfangreichen eigenen Untersuchungen gezeigt, dass in vielen Fällen durch die Adsorption der erfindungsgemäßen Verbindungen an einem, zwei oder sämtlichen der oben genannten Trägerstoffe eine kontrolliertere Freisetzung der Geruchsnote bewirkt wird, verglichen mit Vergleichserzeugnissen, die bei sonst identischer Zusammensetzung keine Trägerstoffe der Komponente (c) enthielten.

Weitere bevorzugte erfindungsgemäße Erzeugnisse umfassen
(d) ein, zwei oder mehr Verbindungen ausgewählt aus der Gruppe bestehend aus Wasser, Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat und Triacetin.

Verbindungen gemäß Komponente (d) dienen regelmäßig als Lösungsmittel und/oder Verdünner in einem erfindungsgemäßen Erzeugnis (wie oben beschrieben, vorzugsweise wie oben als bevorzugt definiert).

Ein besonders bevorzugtes Lösungsmittel und/oder Mittel zum Verdünnen ist Wasser.

Bevorzugte erfindungsgemäße Erzeugnisse umfassen Wasser in einer Gesamtmenge bis 99,999999 Gew.-%, vorzugsweise 5 bis 80 Gew.-%, bezogen auf das Gesamtgewicht des Erzeugnisses.

Andere bevorzugte erfindungsgemäße Erzeugnisse (wie oben beschrieben, vorzugsweise wie oben als bevorzugt definiert) umfassen
(e) ein, zwei oder mehr Bestandteile ausgewählt aus der Gruppe bestehend aus
(e1) Tensiden und sonstigen Grund-, Hilfs- und Zusatzstoffen zum Einsatz in parfümierten Erzeugnissen
   sowie
(e2) Grund-, Hilfs- und Zusatzstoffen zum Einsatz in Erzeugnissen für die Ernährung, die Mundpflege oder den Genuss.

Bei den der Komponente (e) zugeordneten Tensiden (e1), handelt es sich um anionische und/oder nichtionische und/oder amphotere und/oder kationische Tenside.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono-oder Dialkylalkanolamide, Fettsäureester von Polyethylenglykolen, ethoxylierte Fettsäureamide, Alkylpolyglycoside oder Sorbitanetherester geeignet.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder - propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als kationische Tenside eignen sich quaternäre Ammoniumverbindungen, beispielsweise Cetyltrimethylammoniumchlorid.

Besonders bevorzugte erfindungsgemäße Erzeugnisse umfassen ein, zwei oder mehrere Tenside. Insbesondere umfassen solche Erzeugnisse vorzugsweise eine der folgenden Kombinationen der jeweils genannten Komponenten:
(i) Komponente (a) und ein, zwei oder mehrere Tenside, vorzugsweise in weiterer Kombination mit Wasser,
(ii) Komponenten (a), (b) und ein, zwei oder mehrere Tenside, vorzugsweise in weiterer Kombination mit Wasser,
(iii) Komponenten (a), (b), (d) und ein, zwei oder mehrere Tenside.

Bei den der Komponente (e) zugeordneten Grund-, Hilfs- und Zusatzstoffen zum Einsatz in Erzeugnissen für die Ernährung, die Mundpflege oder den Genuss (e2), handelt es sich vorzugsweise um Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. g-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phen-oxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Die vorstehend genannten Grund-, Hilfs- und Zusatzstoffen zum Einsatz in Erzeugnissen für die Ernährung, die Mundpflege oder den Genuss (e2) werden in bevorzugten erfindungsgemäßen Erzeugnissen in einer Gesamtmenge von 5 bis 99,999999 Gew.-%, vorzugsweise 10 bis 80 Gew.-% oder 10 bis 50 Gew.-%, bezogen auf die Gesamtmenge des Erzeugnisses, eingesetzt.

Bei den vorstehend genannten, bevorzugten erfindungsgemäßen Erzeugnissen (wie oben beschrieben, vorzugsweise wie oben als bevorzugt definiert) kann es sich um eine breite Palette von Produkten handeln. So sind bevorzugte erfindungsgemäße Erzeugnisse Parfüm-Extraits, Eau de Parfums, Eau de Toilettes, Rasierwässer, Eau de Colognes, Pre-shave-Produkte, Splash-Colognes und parfümierte Erfrischungstücher sowie parfümierte saure, alkalische und neutrale Reinigungsmittel, wie z.B. Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, Textilerfrischer, Bügelhilfen, flüssige Waschmittel, pulverförmige Waschmittel, Wäschevorbehandlungsmittel wie Bleichmittel, Einweichmittel und Fleckenentferner, Wäscheweichspüler, Waschseifen, Waschtabletten, Desinfektionsmittel, Oberflächendesinfektionsmittel sowie Luftverbesserer in flüssiger, gelartiger oder auf einem festen Träger aufgebrachter Form, Aerosolsprays, Wachse und Polituren wie Möbelpolituren, Fußbodenwachse, Schuhcremes sowie Körperpflegemittel wie z.B. feste und flüssige Seifen, Duschgele, Shampoos, Rasierseifen, Rasierschäume, Badeöle, kosmetische Emulsionen von Öl-in-Wasser-, von Wasser-in-ÖI- und von Wasser-in-ÖI-in-Wasser-Typ wie z.B. Hautcremes- und -lotionen, Gesichtscremes und -lotionen, Sonnenschutzcremes-und -lotionen, Aftersun-cremes und -lotionen, Handcremes und -lotionen, Fußcremes und -lotionen, Enthaarungscremes und -lotionen, After-shave-Cremes und -lotionen, Bräunungscremes und - lotionen, Haarpflegeprodukte wie z.B. Haarsprays, Haargele, festigende Haarlotionen, Haarspülungen, permanente und semipermanente Haarfärbemittel, Haarverformungsmittel wie Kaltwellen und Haarglättungsmittel, Haarwässern, Haarcremes und -lotionen, Deodorantien und Antiperspirantien wie z.B. Achselsprays, Roll-ons, Deosticks, Deocremes, Produkte der dekorativen Kosmetik wie z.B. Lidschatten, Nagellacke, Makeups, Lippenstifte, Mascara sowie Kerzen, Lampenöle, Räucherstäbchen, Insektizide, Repellentien und Treibstoffe.

Besonders bevorzugte erfindungsgemäße Erzeugnisse (wie oben beschrieben, vorzugsweise wie oben als bevorzugt definiert) sind ausgewählt aus der Gruppe bestehend aus Shampoos, Parfüm-Extraits, Eau de Parfums, Eau de Toilettes, Rasierwässern, Eau de Colognes, Pre-shave-Produkten, Splash-Colognes, parfümierten Erfrischungstüchern, sauren, alkalischen und neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachsen und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

Insbesondere in Verbindung mit den zuvor genannten bevorzugten, erfindungsgemäßen Erzeugnissen wurden besonders ausgeprägte positive Sekundäreigenschaften beobachtet (siehe oben im Text). Entsprechend gilt das oben Gesagte zu erfindungsgemäßen Verbindungen (wie oben beschrieben, vorzugsweise wie oben als bevorzugt definiert), erfindungsgemäßen Mischungen (wie oben beschrieben, vorzugsweise wie oben als bevorzugt definiert) und erfindungsgemäßen Erzeugnissen (wie oben beschrieben, vorzugsweise wie oben als bevorzugt definiert).

In einer bevorzugten Ausführungsform liegen erfindungsgemäße Erzeugnisse mikroverkapselt, sprühgetrocknet, als Einschluss-Komplexe oder als Extrusions-Produkte vor. Eine solche Ausführungsform ist vorzugsweise dazu geeignet, in anderen parfümierten Erzeugnissen weiterverarbeitet bzw. zu solchen hinzugefügt zu werden.

Die zuvor beschriebene Mikroverkapselung erfindungsgemäßer Erzeugnisse kann beispielsweise durch das sogenannte Koazervationsverfahren mit Hilfe von Kapselmaterialien z.B. aus polyurethanartigen Stoffen oder Weichgelatine erfolgen. Die sprühgetrockneten Erzeugnisse können beispielsweise durch Sprühtrocknung einer Emulsion bzw. Dispersion (jeweils umfassend erfindungsgemäße Verbindungen) hergestellt werden, wobei als Trägerstoffe modifizierte Stärken, Proteine, Dextrin und pflanzliche Gummen verwendet werden können. Einschluss-Komplexe können z.B. durch Eintragen von Dispersionen (vorzugsweise umfassend erfindungsgemäße Verbindungen und Cyclodextrine oder Harnstoffderivate) in ein geeignetes Lösungsmittel, z.B. Wasser, hergestellt werden. Extrusions-Produkte können durch Verschmelzen erfindungsgemäßer Verbindungen mit einem geeigneten wachsartigen Stoff und/oder durch Extrusion mit nachfolgender Erstarrung, (gegebenenfalls in einem geeigneten Lösungsmittel, vorzugsweise Isopropanol), erhalten werden. In eigenen Untersuchungen hat sich gezeigt, dass sprühgetrocknete erfindungsgemäße Erzeugnisse als Halbfertigwaren besonders gut zur Herstellung von weiteren erfindungsgemäßen Erzeugnissen geeignet sind. Ein solches sprühgetrocknetes Erzeugnis umfasst vorzugweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen, vorzugsweise modifizierte Stärken oder Gummi Arabicum.

Es hat sich in eigenen Untersuchungen gezeigt, dass die Eigenschaften von derartig modifizierten erfindungsgemäßen Erzeugnissen häufig durch sogenanntes Coaten mit geeigneten Materialien (beispielsweise im Hinblick auf eine gezieltere Duftfreisetzung) weiter optimiert werden konnten. Als geeignete Materialien können vorzugsweise wachsartige Kunststoffe, wie z.B. Polyvinylalkohol, verwendet werden.

In einer speziellen alternativen Ausführungsform werden erfindungsgemäße Verbindungen, Mischungen bzw. Erzeugnisse zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. Hinsichtlich der zuvor erwähnten Matrix sind insbesondere solche Matrixmaterialien bevorzugt, die eine verzögerte Freisetzung erfindungsgemäßer Verbindungen, Mischungen oder Erzeugnisse bewirken, so dass eine langanhaltende Wirkung erzielt wird. Besonders bevorzugt ist insoweit eine Fett-, Wachs-, Polysaccharid- oder Proteinmatrix.

In einer anderen speziellen Ausführungsform werden erfindungsgemäße Verbindungen, Mischungen bzw. Erzeugnisse zunächst mit einem, zwei oder mehreren Komplexbildnern, beispielsweise mit Cyclodextrinen oder Cyclodextrinderivaten, bevorzugt alpha- oder beta-Cyclodextrin, komplexiert und in dieser komplexierten Form eingesetzt bzw. weiterverarbeitet.

In einer bevorzugten Ausführungsform betrifft die vorliegende Erfindung der Mundpflege dienende Erzeugnisse, vorzugsweise Mund- und/oder Zahnpflegemittel wie Zahnpasten, Zahngele, Zahnpulver, Mundwässer und andere Mundpflegemittel.

Zahnpflegemittel (der Mundpflege dienende Erzeugnisse), die erfindungsgemäße Verbindungen, Mischungen oder Erzeugnisse umfassen, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite®, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthan-carbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Ein weiterer Aspekt der vorliegenden Erfindung betrifft die Verwendung einer erfindungsgemäßen Verbindung (wie oben beschrieben, vorzugsweise wie oben als bevorzugt definiert) oder einer erfindungsgemäßen Mischung (wie oben beschrieben, vorzugsweise wie oben als bevorzugt definiert) oder eines erfindungsgemäßen Erzeugnisses (wie oben beschrieben, vorzugsweise wie oben als bevorzugt definiert)
- als Riechstoff bzw. als Riechstoffmischung bzw. als Riechstofferzeugnis, vorzugsweise mit einer oder mehreren Geruchsnoten ausgewählt aus der Gruppe bestehend aus moschusartig, strahlend, weich, holzig, erogen, blumig und exaltierend, bevorzugt mit der Geruchsnote moschusartig
   und/oder
- zum Ersetzen eines Moschusriechstoffs, einer Moschusriechstoffmischung oder eines Moschusriechstofferzeugnisses mit geringerem Haftungsvermögen und/oder geringerer Substantivität und/oder geringerer Bioabbaubarkeit
   und/oder
- zum Verstärken und/oder Modifizieren einer Geruchsnote, vorzugsweise einer Geruchsnote ausgewählt aus der Gruppe bestehend aus moschusartig, strahlend, weich, holzig, erogen, blumig und exaltierend, bevorzugt mit der Geruchsnote moschusartig
   und/oder
- zum Versehen von Haut und/oder textilen Fasern mit einer Geruchsnote oder mehreren Geruchsnoten ausgewählt aus der Gruppe bestehend aus moschusartig, strahlend, weich, holzig, erogen, blumig und exaltierend, bevorzugt mit der Geruchsnote moschusartig
   und/oder
- als Mittel zum Erhöhen der Substantivität und/oder Retention einer Riechstoffmischung und/oder als Fixateur und/oder als Mittel zum Erhöhen des über einer tensidhaltigen wässrigen Lösung wahrgenommenen Geruches anderer Riechstoffe.

Es hat sich in eigenen Untersuchungen gezeigt, dass die erfindungsgemäße Verwendung erfindungsgemäßer Verbindungen, erfindungsgemäßer Mischungen und erfindungsgemäßer Erzeugnisse (jeweils wie oben beschrieben, vorzugsweise wie oben als bevorzugt definiert) in vielen Fällen zu einer sehr positiven Ausprägung der (oben beschriebenen) Sekundäreigenschaften führt. Das oben Gesagte gilt dementsprechend. Es hat sich auch gezeigt, dass die Verwendung zu einer positiven Vermittlung von einer, zwei, mehreren oder sämtlichen der Geruchsnoten Moschus, strahlend, weich, holzig, erogen, blumig und exaltierend, vorzugsweise Moschus, führt.

Das oben Gesagte zu erfindungsgemäßen Verbindungen, erfindungsgemäßen Mischungen und erfindungsgemäßen Erzeugnissen (jeweils wie oben beschrieben, vorzugsweise wie jeweils oben als bevorzugt definiert) gilt auch hinsichtlich der erfindungsgemäßen Verwendung.

Es hat sich in eigenen Untersuchungen häufig gezeigt, dass sich die erfindungsgemäßen Verbindungen bzw. erfindungsgemäßen Mischungen hervorragend als sogenannte Booster (Verstärker; Enhancer) eignen. Dabei werden diese Booster bevorzugt in erfindungsgemäßen Mischungen eingesetzt (es gilt das oben Gesagte zu erfindungsgemäßen Verbindungen, Mischungen und Erzeugnissen).

Der Begriff "textile Fasern" umfasst sämtliche Fasern, die sich textil verarbeiten lassen. Gemeinsam ist ihnen eine im Vergleich zu ihrem Querschnitt große Länge sowie ausreichende Festigkeit und Biegsamkeit. Insbesondere umfasst der Begriff "textile Fasern" Naturfasern (wie z.B. Haare) und Chemiefasern.

Zu den Begriffen Substantivität, Haftungsvermögen und Retention siehe die Ausführungen weiter oben. Sofern die vorstehende erfindungsgemäße Verwendung das Ersetzen eines Moschusriechstoffs, einer Moschusriechstoffmischung oder eines Moschusriechstofferzeugnisses mit geringerem Haftungsvermögen und/oder geringerer Substantivität und/oder geringerer Bioabbaubarkeit betrifft, so handelt es sich bei dem zuvor genannten Moschusriechstoff, der zuvor genannten Moschusriechstoffmischung bzw, dem zuvor genannten Moschusriechstofferzeugniss um ein(e) nicht erfindungsgemäße(s) Verbindung, Mischung bzw. Erzeugnis, welche jedoch allesamt die Geruchsnote Moschus vermitteln. Zu den nicht erfindungsgemäßen Verbindungen, die in nicht erfindungsgemäßen Mischungen bzw. Erzeugnissen enthalten sein können, siehe weiter oben im Text.

Die erfindungsgemäße Verwendung (wie oben beschrieben) führt regelmäßig zu positiven fixierenden Eigenschaften, d.h. die erfindungsgemäßen Verbindungen wirken regelmäßig als Fixateur. Als ein Fixateur erhöhen die erfindungsgemäßen Verbindungen (wie oben beschrieben, vorzugsweise wie oben als bevorzugt definiert) die Haftfestigkeit von anderen Riechstoffen, sei es durch deren Dampfdruckerniedrigung oder geruchliche Verstärkung (z.B. Absenkung des Schwellenwertes). Die Erfindung betrifft daher auch die Verwendung der erfindungsgemäßen Verbindungen bzw. Mischungen und Erzeugnisse als Fixateur.

Ein weiterer Aspekt der vorliegenden Erfindung betrifft ein Verfahren zum Vermitteln eines Geruches mit einer, mehreren oder sämtlichen der Geruchsnoten moschusartig, strahlend, weich, holzig, erogen, blumig und exaltierend oder zum Verstärken eines Geruches mit einer, mehreren oder sämtlichen der Geruchsnoten holzig, blumig, frisch, grün, fruchtig, moschusartig und würzig-balsamisch, umfassend die folgenden Schritte:
(i) Herstellen oder Bereitstellen eines oder mehrerer Erzeugnisse, die keine erfindungsgemäße Verbindung umfassen,
   oder
   Bereitstellen von menschlicher oder tierischer Haut oder von menschlichen oder tierischen Haaren,
(ii) Herstellen oder Bereitstellen
   - einer erfindungsgemäßen Verbindung (wie oben beschrieben, vorzugsweise wie oben als bevorzugt bezeichnet),
      oder
   - einer erfindungsgemäßen Mischung (wie oben beschrieben, vorzugsweise wie oben als bevorzugt bezeichnet),
      oder
   - eines erfindungsgemäßen Erzeugnisses (wie oben beschrieben, vorzugsweise wie oben als bevorzugt bezeichnet)
(iii) Vermischen oder Kontaktieren der in den Schritten (i) und (ii) hergestellten oder bereitgestellten Gegenstände, d.h. der in Schritt (i) her- oder bereitgestellten Erzeugnisse bzw. der menschlichen oder tierischen Haut bzw. der menschlichen oder tierischen Haare und der in Schritt (ii) her- oder bereitgestellten erfindungsgemäßen Verbindung oder Mischung oder des erfindungsgemäßen Erzeugnisses.
wobei die in Schritt (iii) eingesetzte Menge des in Schritt (ii) hergestellten Gegenstands ausreicht, eine, mehrere oder sämtliche der Geruchsnoten moschusartig, strahlend, weich, holzig, erogen, blumig und exaltierend zu vermitteln und/oder eine, mehrere oder sämtliche der Geruchsnoten holzig, blumig, frisch, grün, fruchtig, moschusartig und würzig-balsamisch zu verstärken.

Ein weiterer Aspekt der vorliegenden Erfindung betrifft ein Verfahren zur Herstellung einer Verbindung der Formel (la) oder (Ib), umfassend die Schritte:
- Herstellen oder Bereitstellen einer Verbindung der Formel (IV), 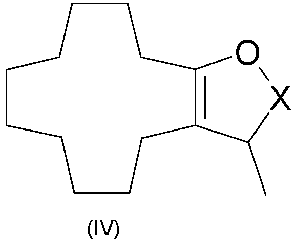 wobei X eine Methylen- oder Ethylen-Gruppe bedeutet
- Umsetzen der Verbindung der Formel (IV) in einem oder mehreren Schritten zur Verbindung der Formel (la) bzw. (Ib).

Die Herstellung erfindungsgemäßer Verbindungen kann mittels an sich bekannter Reaktionen und Verfahren erfolgen.

Zum Beispiel kann Cyclododecanon mit Propenylacetat zu einer Verbindung der Formel (II) umgesetzt werden und anschließend zu den erfindungsgemäßen Verbindungen der Formel (Ia) bzw. (Ib) (X = Methylen) (siehe Formelschema I sowie die Beispiele weiter unten im Text).

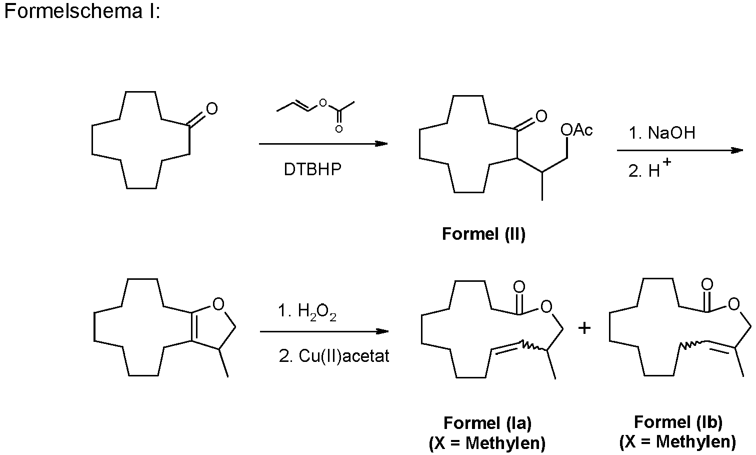

Angelehnt an die Lehre von DE 3137939 A1 kann Cyclododecanon zu einer Verbindung der Formel (III) umgesetzt werden. Ausgehend von einer Verbindung der Formel (III) kann diese Verbindung in weiteren Reaktionsschritten zu erfindungsgemäßen Verbindungen der Formel (Ia) bzw. (Ib) mit X = Methylen umgesetzt werden (siehe Formelschema II sowie die Beispiele weiter unten im Text).

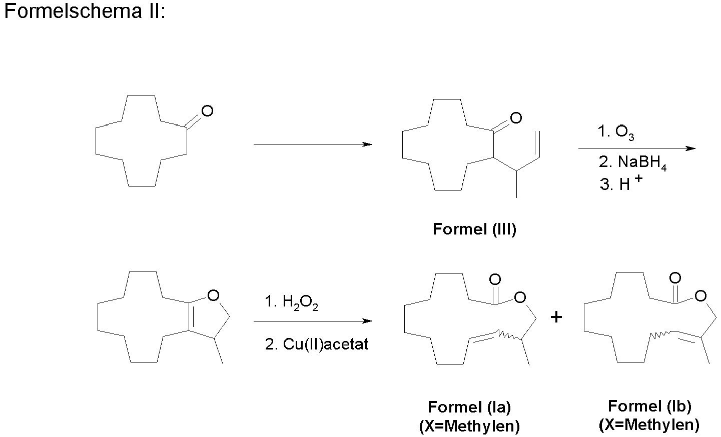

In alternativen Reaktionsschritten, ausgehend von einer Verbindung der Formel (III), kann diese Verbindung auch zu erfindungsgemäßen Verbindung der Formel (Ia) bzw. (Ib) mit X= Ethylen umgesetzt werden (siehe Formelschema III):

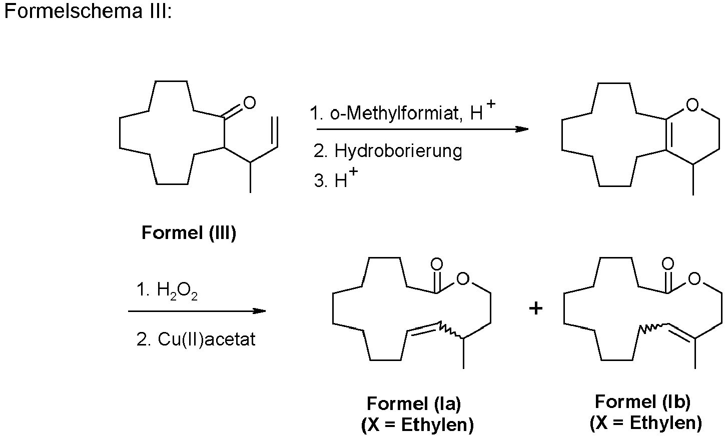

Dem Fachmann sind geeignete Methoden bzw. Verfahren bekannt, die es erlauben, die erfindungsgemäßen Verbindungen der Formel (Ia) bzw. (Ib) voneinander zu trennen, sofern sie nach der Synthese in einem Reaktionsgemisch (und gegebenenfalls in unterschiedlichen Konzentrationen) vorliegen. Dazu eignen sich übliche Trennverfahren, vorzugsweise HPLC- und/oder PCG-Verfahren unter Anwendung geeigneter Verfahrensbedingungen.

Ein weiterer Aspekt der vorliegenden Erfindung betrifft die Verwendung einer Verbindung der Formel (IV) 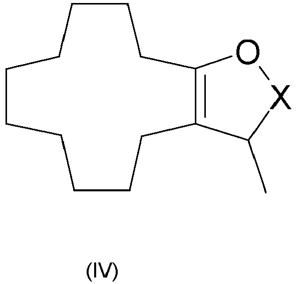 wobei X eine Methylen- oder Ethylen-Gruppe bedeutet
als Intermediat bei der Herstellung einer erfindungsgemäßen Verbindung der Formel (la) oder (Ib).

Die folgenden Beispiele erläutern exemplarisch die Erfindung. Sofern nicht anders angegeben, beziehen sich alle Daten, insbesondere die %-Angaben, auf das Gewicht.

### Beispiele:

### (A) Synthesebeispiele:

In den nachfolgenden Beispielen haben die nachfolgend aufgezählten Abkürzungen die folgenden Bedeutungen:
d. Th.: der Theorie
Sdp.: Siedepunkt (unter Normalbedingungen, sofern nicht anders angegeben)
E/Z Gemisch: Prozentualer Anteil an E- bzw. Z-konfigurierten Reaktionsprodukten
MS: Ergebnis der massenspektrometrischen Untersuchung

### Synthesebeispiel 1: Herstellung von Prop-1-enylacetat :

In einem 2 l Rührwerk wurden 215 g (3,6 mol) Propionaldehyd, 1530 g (15 mol) Essigsäureanhydrid und 36 g Kaliumacetat vorgelegt und langsam aufgeheizt. Anschließend wurde für die Dauer von 8 Stunden unter Rückfluss nachgerührt. Danach wurde die Reaktionslösung an einer 30 cm Füllkörper-Kolonne unter Normaldruck fraktioniert.
Ausbeute: 249 g Prop-1-enyl-acetat (66 % d. Th.)
Sdp.: 69°-120°C
GC-Auswertung (20m ZB-WAX, Innendurchmesser 0,18µm / 60-9-220°C Kaltaufgabesystem)
E/Z Gemisch 53:47
MS: m/z: 29 (8), 39 (6), 43 (100), 57 (15), 58 (13), 72(0,2), 82(0,2), 100 (13, M+)

### Synthesebeispiel 2: Herstellung von 2-(2-oxocyclododecyl)propylacetat (Verbindung der Formel (II)):

In einem 1 l Rührwerk wurden 560 g (3,07 mol) Cyclododecanon und 1,9 g Aluminiumtriisopropylat vorgelegt und auf 160°C erhitzt. Anschließend wurde innerhalb von 5 Stunden ein Gemisch aus 118 g (1,18 mol) Prop-1-enylacetat (siehe Synthesebeispiel 1) und 16 g Di-tert.-Butylperoxid bei 160°C unter Rühren dosiert zugegeben und weitere 2 Stunden bei ebenfalls 160°C nachgerührt. Anschließend wurde an einer 20 cm Vigreux-Kolonne im Vakuum aus der Reaktionslösung Cyclododecanon abdestilliert.
Ausbeute: 120 g
2-(2-oxocyclododecyl)propylacetat als Rückstand (36% d. Th.)
GC-Auswertung (20m ZB-WAX, Innendurchmesser 0,18µm / 60-9-220°C Kaltaufgabesystem)
2-(2-oxocyclododecyl)propylacetat: Diastereomer I (34%) u. Diastereomer II (34,5%)
MS: m/z: 43 (100), 55 (76), 69 (38), 81 (30), 98 (68), 111 (45), 137(10), 151(15), 165(12), 207(21), 222(21), 267(0,4), 282(0,8, M+)
und Nebenprodukt (29%)
1-(2-oxocyclododecyl)propylacetat
MS: m/z: 43 (100), 55 (57), 67 (43), 81 (34), 98 (35), 111 (15), 137(10), 151(8), 165(6), 193(19), 222(20), 267(0,4), 282(0,8, M+)

### Synthesebeispiel 3: Herstellung von 3-Methyl-2,3,4,5,6,7,8,9,10,11,12,13-dodecahydro-cyclododeca[b]furan (nicht erfindungsgemäß):

In einen 1 l Rührwerk wurden 120 g (0,42 mol) 2-(2-oxocyclododecyl)propylacetat (Formel (II), siehe Synthesebeispiel 2), 250 g Methanol und 77 g (0,56 mol) Natronlauge (32%ig) vorgelegt und 2 Stunden unter Rückfluss gesiedet. Anschließend wurde das Methanol abdestilliert und Toluol zum Reaktionsansatz hinzugegeben. In einem nachfolgenden Schritt wurde das Reaktionsgemisch ein Mal mit 5%iger Schwefelsäure gewaschen, mit 0,5 g para-Toluolsulfonsäure versetzt und 1 Stunde am Wasserabscheider gekocht. Danach wurden 0,5 g Natriumcarbonat zugeben, das Toluol abdestilliert und die Rohausbeute (142 g) an einer 20 cm Vigreux-Kolonne unter Vakuum fraktioniert.
Ausbeute: 65 g 3-Methyl-2,3,4,5,6,7,8,9,10,11,12,13-dodecahydro-cyclododeca[b]furan (24,8% d. Th. bezogen auf eingesetztes Prop-1-enyl-acetat)
MS: m/z: 41 (99), 55 (94), 67 (63), 81 (45), 98 (100), 111(97), 123(82), 137 (21), 151(36), 165(28), 179(18), 193(4), 207(72), 222(60, M+)

### Synthesebeispiel 4: Herstellung von (E,Z)-14-Methyl-1-oxacyclopentadec-12/13-en-2-on (erfindungsgemäß):

### 1. Stufe: Hydroperoxidierung:

In einem 1 l Rührwerk wurden 65 g (0,29 mol) 3-Methyl-2,3,4,5,6,7,8,9,10,11,12,13-dodecahydrocyclododeca[b]furan und 234 g Methylisobutylketon vorgelegt und auf 0°C abgekühlt. Unter Rühren und Kühlen bei einer Temperatur von 0°C wurden 22 g 70%ige Schwefelsäure und 36 g Perhydrol (30%ig) dosiert zugegeben und das Reaktionsgemisch anschließend noch 15 Min. gerührt. In einem weiteren Schritt wurde das Reaktionsgemisch mit 240 g Natronlauge (5%ig) 15 Min. gerührt.

### Ausbeute: 312 g Hydroperoxid-Lsg.

### 2. Stufe: Spaltung des Hydroperoxides:

In einem 1 l Rührwerk mit Wasserabscheider wurden 117 g Methylisobutylketon und 2,9 g Kupfer(II)-acetat zum Sieden erhitzt. Dann wurde die Hydroperoxid-Lsg. (vergleiche 1. Stufe: 312 g) innerhalb von 1 Stunde unter Rückfluss zugetropft und anschließend 1 Stunde unter Rückfluss nachgerührt. Das Reaktionsgemisch wurde anschließend filtriert, mit Natriumsulfit-Lsg. (5%ig) gemischt und gerührt, mit Kochsalz-Lösung neutral gewaschen und in einem nachfolgenden Schritt eingedampft. Die Rohausbeute von 71 g wurde an einer 20 cm Vigreux-Kolonne unter Vakuum fraktioniert.
Ausbeute: 57,8 g
(E,Z)-14-Methyl-1-oxacyclopentadec-12-en-2-on (53%) (Verbindung der Formel (la) mit X = CH_{2,} R- und S-konfiguriert und E- und Z-konfiguriert,
(E,Z)-14-Methyl-1-oxacyclopentadec-13-en-2-on (18%) (Verbindung der Formel (Ib) mit X = CH_{2,} E- und Z-konfiguriert),
14-Methyl-1-oxacyclopentadecan-2-on (18,9%) (nicht erfindungsgemäß)
und
2-Propylidencyclododecanon (10%) (nicht erfindungsgemäß)
Sdp.: 170°-190°C/ 0,9 mbar

### MS-Analyse:

### (E,Z)-14-Methyl-1-oxacyclopentadec-12/13-en-2-on (erfindungsgemäße Verbindungen):

MS: m/z: 41 (90), 55 (79), 67 (76), 82 (100), 95 (72), 109 (18), 123 (8), 135 (5), 149 (2), 164 (8), 182 (4), 195 (30), 223 (3), 238 (9, M+)

### 14-Methyl-1-oxacyclopentadecan-2-on (nicht erfindungsgemäß):

MS: m/z: 41 (95), 55 (100), 69 (56), 83 (36), 97 (29), 111 (14), 125 (7), 138 (6), 149 (4), 167 (6), 180 (3), 197 (1), 222 (6), 240 (1, M+)

Die ungesättigten Lactone lassen sich durch Destillation oder chromatographische Methoden auf über 98% anreichern.

### Synthesebeispiel 5: Herstellung von 1,1-Dimethoxycyclododecan:

In einem 2 l Rührwerk wurden 524 g (4,94 mol) Trimethylorthoformiat, 132 g (4,13 mol) Methanol und 3 g para-Toluolsulfonsäure vorgelegt und auf 45°C erwärmt. Danach wurden 375 g (2,06 mol) Cyclododecanon langsam unter Rühren zugetropft. Anschließend wurde das Reaktionsgemisch für die Dauer von 4 Stunden unter Rückfluss gekocht, auf Raumtemperatur abgekühlt, mit 250 ml tert.-Butylmethylether verdünnt und anschließend zwei Mal mit je 300 ml 5%iger Soda-Lösung gewaschen. Die organische Phase wurde eingedampft. Die Rohausbeute betrug 475 g Rohprodukt, welches an einer 30 cm-Füllkörperkolonne über 0,5 g Natriumcarbonat fraktioniert wurde.
Ausbeute: 306 g 1,1-dimethoxycyclododecan (63% d.Th.)
Sdp.: 110-114°C/1,3 mbar.
GC-Auswertung: 20 m DB-1 Innendurchmesser 0.18µm / 60-50-240°
MS: m/z: 29 (7), 41 (14), 55 (18), 72 (15), 88 (25), 101 (100), 125 (5), 157 (5), 197 (21), 228 (2, M+)

### Synthesebeispiel 6: Herstellung von 2-(1-Methylallyl)cyclododecanon (Verbindung der Formel (III)):

In einem 2 l Rührwerk mit 30 cm-Füllkörperkolonne und Kolonnenkopf wurden 50 g (0,21 mol) 1,1-Dimethoxycyclododecan (97%ig, siehe Synthesebeispiel 5), 432 g (5,4 mol) Crotylalkohol (90%ig, cis/trans-Gemisch) und 8 g Propionsäure aufgeheizt. Die Methanolabspaltung setzte bei ca. 88°C ein. Es wurden 458 g (1,95 mol) 1,1-dimethoxycyclododecan (97%ig, siehe Synthesebeispiel 5) während einer Dauer von 2,5 Stunden bei 111-138°C im Sumpf und paralleler Destillatabnahme (Kopftemperatur: 64-65°C, Rücklaufverhältnis: 5:1, Destillat: 85 g) kontrolliert zugetropft. Anschließend wurde während der Dauer von etwa 4 Stunden bis zu einer Sumpftemperatur von 151°C ein Gemisch von Methanol und Crotylalkohol (Kopftemperatur: 65-120°C) abdestilliert, noch 1h bei 150-151°C gerührt und auf Raumtemperatur abgekühlt. Bei Raumtemperatur wurden dem Reaktionsgemisch 500 ml MTBE (Methyl-tert-butylether) zugegeben, das Reaktionsgemisch anschließend mit 250 ml 5%iger Soda-Lösung gewaschen und danach eingedampft. Die Ausbeute des Rohproduktes betrug 530 g. Das Rohprodukt wurde anschließend an einer 30 cm-Füllkörperkolonne fraktioniert.
Ausbeute: 436 g 2-(1-Methylallyl)cyclododecanon (85% d.Th.)
Sdp.: 108-111°C/1mbar.
GC-Auswertung: 20m DB-1 Innendurchmesser 0.18µm / 60-50-240°
Summe der Isomeren: 99,7%
MS: m/z: 41 (54), 55 (100), 67 (53), 81 (55), 95 (46), 112 (25), 125 (21), 149 (8), 221 (11), 236 (10, M+)

### Synthesebeispiel 7: Herstellung von (1Z)-1-Methoxy-12-(1-methylallyl)cyclododecen:

In einem 2 l Rührwerk wurden unter Stickstoffatmosphäre 390 g (3,68 mol) Trimethylortho-formiat, 235 g (7,34 mol) Methanol und 6 g para-Toluolsulfonsäure vorgelegt. Unter Rückfluss wurden 436 g (1,84 mol) 2-(1-Methylallyl)cyclododecanon (siehe Synthesebeispiel 6) innerhalb von 1,5 Stunden kontrolliert zugetropft. Anschließend wurde während einer Dauer von 17 Stunden unter Rückfluss nachgerührt. In einem nächsten Schritt wurde das Reaktionsgemisch unter kräftigem Rühren mit 500 ml 5%ige Soda-Lösung vermischt, mit 250 ml Toluol (als Extraktionsmittel) verrührt und die extrahierte organische Phase ein Mal mit 250 ml 5%iger Soda-Lösung gewaschen. Nach dem Eindampfen der gewaschenen Lösung (60-80°C/300-20 mbar) wurden 472 g 88%iges Rohprodukt erhalten. Dieses Rohprodukt wurde an einer 30 cm-Füllkörperkolonne fraktioniert destilliert.
Ausbeute: 195 g (1Z)-1-Methoxy-12-(1-methylallyl)cyclododecen (41% d.Th. ; farblose Flüssigkeit)
Sdp.: 112-114°C/1mbar.
GC-Auswertung: 20m DB-1 Innendurchmesser 0.18µm / 60-50-240°
Summe der Isomeren: 95,7%
MS: m/z: 41 (56), 55 (76), 67 (89), 81 (100), 95 (64), 111 (95), 125 (40), 139 (26), 163 (15), 195 (8), 235 (7), 250 (22, M+)

### Synthesebeispiel 8: Herstellung von 4-Methyl-3,4,5,6,7,8,9,10,11,12,13,14-dodecahydro-2H-cyclo-dodeca[b]pyran:

### 1. Stufe: Hydroborierung :

In einem 0,5 l Rührwerk wurden unter Stickstoffatmosphäre 35,2 g (0,14 mol) (1Z)-1-Methoxy-12-(1-methylallyl)cyclododecen (siehe Synthesebeispiel 7) in 127 ml Tetrahydrofuran bei RT vorgelegt. Anschließend wurden 50 ml (50 mmol) Boran-THF-Komplex (1M in THF) innerhalb von 1 bis 1,5h unter leichter Kühlung bei Raumtemperatur kontrolliert zugetropft und das so erhaltene Reaktionsgemisch für die Dauer einer weiteren Stunde nachgerührt. Anschließend wurde die Reaktionslösung mit 12,7 ml Wasser und 16,7 g 12%ige Natronlauge langsam versetzt. Danach wurden 18,3 g (162 mmol) Wasserstoffperoxid 30%ig innerhalb von 1,5h bei 30-50°C kontrolliert zugetropft und das so erhaltene Reaktionsgemisch für die Dauer einer weiteren Stunde bei RT gerührt. Zur Aufarbeitung wurden 200 ml Toluol und 300 ml Wasser zugegeben, die Phasen voneinander getrennt, die organische Phase zwei Mal mit je 100 ml 5%iger NaCl-Lösung gewaschen und mit 200 ml 5%iger Natriumthiosulfatlösung ausgerührt. Die abgetrennte organische Phase wurde anschließend eingedampft, der so erhaltene Rückstand in 380 ml Aceton gelöst und über Nacht nach Zugabe von 144 g 10%iger Schwefelsäure gerührt. In einem nächsten Schritt wurde das Aceton unter Vakuum abdestilliert, der Rückstand mit Toluol versetzt und anschließend mit 5%iger NaCI-Lösung gewaschen. Es wurden 270 g 2-(3-Hydroxy-1-methylpropyl)cyclododecanon als toluolische Lösung (5%ig) erhalten, die ohne weitere Bearbeitung für die nächste Synthesestufe eingesetzt wurden.
GC-Auswertung: 20m DB-1 Innendurchmesser 0.18µm / 60-50-240°
MS: m/z: 41 (66), 55 (100), 69 (42), 81 (48), 95 (29), 112 (18), 130 (18), 209 (6), 236 (3), 254 (4, M+)

### 2. Stufe: Cyclisierung :

In einem 0,5 l Rührwerk mit Wasserabscheider wurden 270 g toluolische Lösung (erhalten nach der 1. Stufe)und 1,5 g para-Toluolsulfonsäure für die Dauer von 2,5h am Wasserabscheider gerührt und gekocht (abgeschiedenes Wasser: 1,4 ml). Das Reaktionsgemisch wurde anschließend bei RT mit 5%iger Soda-Lösung und mit 5%iger NaCI-Lösung gewaschen. Nach Abziehen des Toluols wurden 36 g Rohprodukt erhalten, destilliert an einer Kugelrohrdestillation bei 140-180°C und 0,8mbar.
Ausbeute: 31 g 4-Methyl-3,4,5,6,7,8,9,10,11,12,13,14-dodecahydro-2H-cyclo-dodeca[b]pyran.
GC-Auswertung: 20m DB-1 Innendurchmesser 0.18µm / 60-50-240°
MS: m/z: 41 (35), 55 (35), 67 (19), 81 (17), 97 (15), 112 (100), 125 (33), 137 (24), 165 (12), 221 (24), 236 (25, M+)

### Synthesebeispiel 9: Herstellung von (12E/Z)-14-Methyl-1-oxacyclohexadec-12-en-2-on (erfindungsgemäß):

### 1. Stufe: Hydroperoxidierung:

In einem 0,250 l Rührwerk wurden 31 g 4-Methyl-3,4,5,6,7,8,9,10,11,12,13,14-dodecahydro-2H-cyclo-dodeca[b]pyran (siehe Synthesebeispiel 8) und 104 g Methylisobutylketon vorgelegt und bei einer Temperatur von 0 bis 5°C kontrolliert mit 11 g (79 mmol) 70%iger Schwefelsäure und 17,8 g (157 mmol) Wasserstoffperoxid (30%ig) versetzt und für weitere 15 Minuten gerührt. Zur Aufarbeitung wurden 53 ml Wasser bei einer Temperatur von 0 bis 10°C zugegeben, das so erhaltene Reaktionsgemisch mit 20%iger Natronlauge auf einen pH-Wert von 6 eingestellt und anschließend mit 5%iger NaCI-Lösung gewaschen.
Ausbeute 160 g Hydroperoxid-Lösung.

### 2. Stufe: Spaltung des Hydroperoxides:

In einem 0,5 l Rührwerk mit Wasserabscheider wurden 52 g Methylisobutylketon und 1,3 g (6,6 mmol) Kupfer(II)-acetat Monohydrat zum Sieden erhitzt. Die Hydroperoxid-Lösung (160 g, erhalten aus der 1. Stufe) wurden innerhalb von 1h bei 115 bis 119°C kontrolliert zugetropft und das so erhaltene Reaktionsgemisch für die Dauer einer weiteren Stunde gekocht. Zur Aufarbeitung des Reaktionsgemisches wurde mit Wasser und mit 5%iger NaCI-Lösung gewaschen, die organische Phase eingedampft und das Rohprodukt (31 g) an einer Kolonne bei 120-170°C/1,3-3,0 mbar destilliert.
GC-Auswertung: 20m DB-1 Innendurchmesser 0.18µm / 60-50-240°
Ausbeute: 28 g
(E/Z)-14-Methyl-1-oxacyclohexadec-12-en-2-on (70,4%) (Verbindung der Formel (Ia) mit X = C₂H₄, R- und S-konfiguriert und E- und Z-konfiguriert,
(E/Z)-14-Methyl-1-oxacyclohexadec-13-en-2-on (23,5%) (Verbindung der Formel (Ib) mit X = C₂H₄, E- und Z-konfiguriert)
und
14-Methyl-1-oxacyclohexadecan-2-on (6,1%) (nicht erfindungsgemäß).

### MS-Auswertung:

### (E,Z)-14-Methyl-1-oxacyclohexadec-12/13-en-2-on (erfindungsgemäße Verbindungen der Formeln (Ia) und (Ib):

MS: m/z: 41 (48), 55 (39), 67 (43), 81 (100), 95 (34), 109 (23), 123 (5), 164 (4), 223 (4), 252 (5, M+)
14-Methyl-1-oxacyclohexadecan-2-on (nicht erfindungsgemäß):
MS: m/z: 41 (85), 55 (88), 70 (100), 81 (63), 97 (32), 111 (53), 165 (46), 207 (5), 238 (13), 254 (1, M+)

Die ungesättigten Lactone lassen sich durch Destillation oder chromatographische Methoden auf über 98% anreichern.

### Synthesebeispiel 10: Herstellung von 3-Methyl-2,3,4,5,6,7,8,9,10,11,12,13-dodecahydrocyclododeca[b]furan:

### 1. Stufe: Ozonolyse:

In einem 2 l Rührwerk mit Gaseinleitung wurden 102 g (0,42 mol) 2-(1-Methylallyl)-cyclododecanon (98%ig) (siehe Synthesebeispiel 6) in 1000 ml Toluol vorgelegt, für die Dauer von 2h bei -10 bis 0°C ozonhaltiger Sauerstoff (Ozongehalt: ca. 51 g O₃/m³) eingeleitet und anschließend für die Dauer von 20 Minuten mit Stickstoff begast. In einem anschließenden Reaktionsschritt wurden 79 g (1,27 mol) Dimethylsulfid in 80 ml Toluol bei -10 bis 0°C innerhalb von 1,5h kontrolliert zugetropft, das so erhaltene Reaktionsgemisch über Nacht bei Raumtemperatur nachgerührt und anschließend mit Wasser und 5%iger NaCI-Lösung gewaschen. In einem weiteren Schritt wurde das Reaktionsgemisch mit 500 ml 5%iger Na₂SO₃-Lösung vermischt, über Nacht gerührt und anschließend mit 5%iger NaCI-Lösung gewaschen. Das Toluol wurde anschließend abdestilliert.
Ausbeute: 88 g 2-(2-Oxocyclododecyl)propanal (GC: 70%, entspricht 62% d.Th.).
GC-Auswertung: 20m DB-1 Innendurchmesser 0.18µm / 60-50-240°
MS: m/z: 41 (100), 55 (98), 69 (57), 81 (36), 98 (36), 111 (22), 125 (11), 149 (4), 181 (6), 209 (10), 238 (5, M⁺)

### 2. Stufe: Reduktion:

In einem 4 l Rührwerk wurden 2260 ml Methanol, 57 g Natriumcarbonat und 113 g 2-(2-Oxocyclododecyl)propanal (erhalten nach der 1. Stufe) vorgelegt. Unter Kühlung (d.h. bei 0 bis 5°C) wurden 3,6 g (0,095 mol) Natriumborhydrid in 58 g 1%iger Natronlauge kontrolliert zugetropft und das erhaltene Reaktionsgemisch für die Dauer von 2h nachgerührt. Das im Reaktionsgemisch enthaltene Methanol wurde bei 60°C/15 mbar abdestilliert, der Rückstand mit Wasser und Toluol versetzt und anschließend mit 5%iger NaCl-Lösung gewaschen.
Ausbeute: 862g 2-(2-Hydroxy-1-methylethyl)cyclododecanon als toluolische Lösung (13%ig).
GC-Auswertung: 20m DB-1 Innendurchmesser 0.18µm / 60-50-240°
MS: m/z: 29 (29), 41 (81), 55 (100), 69 (50) 81 (37), 98 (65), 111 (37), 123 (21), 137 (7), 151 (9), 165 (7), 182 (19), 207 (16), 222 (13), 240 (3, M⁺)

### 3. Stufe: Cyclisierung:

In einem 1 l Rührwerk mit Wasserabscheider wurden 862 g 2-(2-Hydroxy-1-methylethyl)cyclododecanon (13%ig in Toluol, erhalten nach der 2. Stufe) und 4 g para-Toluolsulfonsäure für die Dauer von 2h am Wasserabscheider gekocht. Anschließend wurde die Reaktioslösung mit 5%iger Soda-Lösung gewaschen und das Toluol abdestilliert.
Ausbeute: 45g 3-Methyl-2,3,4,5,6,7,8,9,10,11,12,13-dodecahydrocyclodo-deca[b]furan
Sdp.: 109-124°C/0,9mbar
GC-Auswertung: 20m DB-1 Innendurchmesser 0.18µm / 60-50-240°
MS: m/z: 41 (99,6), 55 (100), 67 (64), 81 (44), 95 (59), 111 (83), 123 (74), 137 (18), 151 (28), 165 (22), 179 (15), 207 (60), 222 (36, M⁺)

### Synthesebeispiel 11: Herstellung von (E,Z)-14-Methyl-1-oxacyclopentadec-12-en-2-on (erfindungsgemäß)

### 1. Stufe: Hydroperoxidierung:

In einem 0,5 l Rührwerk mit Wasserabscheider wurden 45 g (0,16 mol) 3-Methyl-2,3,4,5,6,7,8,9,10,11,12,13-dodecahydrocyclododeca[b]furan (siehe Synthesebeispiel 10) und 162 g Methylisobutylketon vorgelegt. In einem weiteren Schritt wurden bei 0 bis 5°C langsam und kontrolliert 17 g 70%ige Schwefelsäure sowie 28 g Wasserstoffperoxid (30%ig) zugetropft und das so erhaltene Reaktionsgemisch für weitere 15 Minuten gerührt. Zur Aufarbeitung wurden 81 ml Wasser bei 0 bis 10°C zugegeben und das Reaktionsgemisch anschließend durch Zugabe 20%iger Natronlauge auf einen pH-Wert von 6 eingestellt. Anschließend wurde das Reaktionsgemisch mit 5%iger NaCI-Lösung gewaschen.
Ausbeute: 216 g Hydroperoxid-Lösung.

### 2. Sufe:

In einem 1 l Rührwerk mit Wasserabscheider wurden 81 g Methylisobutylketon und 2 g (10 mmol) Kupfer(II)-acetat zum Sieden erhitzt. Die Hydroperoxid-Lösung (216 g, erhalten aus der 1. Stufe) wurde innerhalb einer Stunde unter Rückfluss kontrolliert zugetropft und das erhaltene Reaktionsgemisch für die Dauer einer weiteren Stunde gekocht. Zur Aufarbeitung wurde das Reaktionsgemisch mit 5%iger NaCI-Lösung gewaschen, das gewaschene Reaktionsgemisch über Nacht mit 5%iger Natriumsulfit-Lösung gerührt und anschließend erneut mit 5%iger NaCI-Lösung gewaschen. Die organische Phase wurde in einem weiteren Schritt eingedampft und das Rohprodukt (49 g) an einer kurzen Kolonne bei 170-190°C/0,9 mbar destilliert.
GC-Auswertung: 20m DB-1 Innendurchmesser 0.18µm / 60-50-240°
Ausbeute: 39g
(E,Z)-14-Methyl-1-oxacyclopentadec-12-en-2-on (58,9%) (Verbindung der Formel (la) mit X = CH_{2,} R- und S-konfiguriert und E- und Z-konfiguriert,
(E,Z)-14-Methyl-1-oxacyclopentadec-13-en-2-on (20%) (Verbindung der Formel (Ib) mit X = CH_{2,} E- und Z-konfiguriert)
und
14-Methyl-1-oxacyclopentadecan-2-on (21%) (nicht erfindungsgemäß)

### MS-Analyse :

### (E,Z)-14-Methyl-1-oxacyclopentadec-12/13-en-2-on (erfindungsgemäße Verbindungen der Formel (la) und (Ib):

MS: m/z: 41 (90), 55 (79), 67 (76), 82 (100), 95 (72), 109 (18), 123 (8), 135 (5), 149 (2), 164 (8), 182 (4), 195 (30), 223 (3), 238 (9, M⁺)

### 14-Methyl-1-oxacyclopentadecan-2-on (nicht erfindungsgemäß):

MS: m/z: 41 (95), 55 (100), 69 (56), 83 (36), 97 (29), 111 (14), 125 (7), 138 (6), 149 (4), 167 (6), 180 (3), 197 (1), 222 (6), 240 (1, M⁺)
Die ungesättigten Lactone lassen sich durch Destillation oder chromatographische Methoden auf über 98% anreichern.

### (B) Anwendungsbeispiele:

### Anwendungsbeispiel 1: Parfümkomposition A (Riechstoffkomposition)

| | |
|---|---|
| Agrumex LC | 10.00 |
| Amarocit® 10%ig in DPG | 10.00 |
| Ambroxid krist. | 10.00 |
| Basilikumöl | 10.00 |
| Calone 1951 10%ig in DPG | 10.00 |
| Cedernholzöl | 10.00 |
| Cedrol Krist | 50.00 |
| Citral 10%ig in DPG | 10.00 |
| Citonellol | 5.00 |
| Cumarin | 10.00 |
| Cyclogalbanat® 10%ig in DPG | 15.00 |
| Dihydromyrcenol | 80.00 |
| Farenal® 10%ig in DPG | 5.00 |
| Galbex 10%ig in DPG | 25.00 |
| Geraniol | 80.00 |
| Geranyl nitrile | 40.00 |
| Hedion | 90.00 |
| Helional | 20.00 |
| Heliotropin | 5.00 |
| Hexenol cis-3 10%ig in DPG | 15.00 |
| Hexenylsalicylat cis-3 | 10.00 |
| Beta-Ionon | 5.00 |
| Iso E Super | 180.00 |
| Isodamascon® 10%ig in DPG | 10.00 |
| Isogalbanat | 20.00 |
| Isoraldein 70 | 20.00 |
| Ketamber 10%ig in TEC | 25.00 |
| Lavandinoel Grosso Nat. | 15.00 |
| Lilial | 20.00 |
| Linalool | 20.00 |
| Linalylacetat | 40.00 |
| Mandarinenoel brasil. grün | 50.00 |
| Timberol® | 40.00 |
| Vanillin | 5.00 |
| Veloutone 10%ig in DPG | 20.00 |
| Ysamber K® | 10.00 |
| | |
| Gesamt | 1000.00 |

| | |
|---|---|
| DPG = Dipropylenglycol TEC = Triethylcitrat | |

Geruchsbeschreibung der Parfümkomposition A (d.h. ohne Zusatz von Verbindungen der Formel (la) bzw. (Ib)): frisch, holzig.

Nach Meinung der Parfümeure wird die Parfümkomposition A insbesondere durch den Zusatz von 3 Gew.-% von ungesättigten Lactonen der Formel (la) bzw. (Ib) mit X = Methylen (d.h. jeweils mit X = Methylen) (es resultiert eine modifizierte Parfümkomposition A) frischer, strahlender, abgerundeter und harmonischer, wobei eine moschusartige und süße Note hinzukommt und die holzigen und blumigen Aspekte verstärkt werden. Die eingesetzten ungesättigten Lactone der Formel (la) bzw. (Ib) verleihen der Komposition durch ihren Eigengeruch sowie durch ihre modifizierende und verstärkende Wirkung (Booster-Wirkung) einen eigenen Charakter und verbinden die unterschiedlichen geruchlichen Elemente.

### Anwendungsbeispiel 2: Parfümkomposition B (Riechstoffkomposition)

| | |
|---|---|
| Allylcyclohexylpropionat | 3.00 |
| Amylsalicylat | 2.00 |
| Benzylacetat | 64.00 |
| itronellol | 122.00 |
| Citral 10% ig in DPG | 2.00 |
| Cyclamenaldehyd | 10.00 |
| Dihydromyrcenol | 3.00 |
| Dimethylbenzylcarbinylacetat | 3.00 |
| Ethylsalicylat 10% ig in DPG | 2.00 |
| Eugenol | 3.00 |
| Indoflor 10%ig in DPG | 16.00 |
| Galaxolide 50%ig in DPG | 164.00 |
| Geraniol | 35.00 |
| Dihydromethyljasmonate | 6.00 |
| Heliotropin | 4.00 |
| Hexylzimtaldehyd | 121.00 |
| Vertocitral | 4.00 |
| Hydroxycitronellal | 42.00 |
| Indol | 2.00 |
| Isobutylsalicylat | 6.00 |
| Lavandinoel Grosso Nat. | 6.00 |
| Acetylcedren | 10.00 |
| Lilial | 190.00 |
| Linalool | 35.00 |
| Linalylacetat | 10.00 |
| Methylanthranilat 10%ig in DPG | 5.00 |
| Nerol | 10.00 |
| Orangenoel | 6.00 |
| Phantolide | 4.00 |
| Phenylacetaldehyddimethylacetal | 6.00 |
| Phenylethylalkohol | 75.00 |
| Rosatol 10%ig in DPG | 6.00 |
| Sandelholzoel | 3.00 |
| Sandranol | 16.00 |
| Trifernal | 2.00 |
| Tonalid | 2.00 |
| | |
| Gesamt | 1000.00 |

| | |
|---|---|
| DPG: Dipropylenglycol | |

Geruchsbeschreibung der Parfümkomposition B (d.h. ohne Zusatz von Verbindungen der Formel (la) bzw. (Ib)): blumig, Maiglöckchen-Geruch.

Nach Meinung der Parfümeure erwacht die Parfümkomposition B insbesondere durch den Zusatz von 6 Gew.-% von Verbindungen der Formel (la) bzw. (Ib) mit X = Ethylen (d.h. jeweils mit X = Ethylen) (es resultiert eine modifizierte Parfümkomposition B) zu neuem Leben. Der Eindruck von Blumigkeit wird erheblich verstärkt. Die Komposition wirkt strahlender, abgerundeter und harmonischer, wobei eine moschusartige und natürliche Note hinzukommt. Die eingesetzten ungesättigten Lactone der Formel (la) bzw. (Ib) (mit jeweils X = Ethylen) verleihen der Komposition durch ihren Eigengeruch sowie durch ihre modifizierende und verstärkende Wirkung (Booster-Wirkung) einen eigenen Charakter und verbinden die unterschiedlichen geruchlichen Elemente.

### Anwendungsbeispiel 3: Shampoo

Verbindungen der Formel (la) bzw. (Ib) mit X = Methylen (d.h. jeweils mit X = Methylen) wurden in einer Gesamtmenge von 0,5 Gew.-% in eine Shampoo-Grundmasse folgender Zusammensetzung eingearbeitet:

| | |
|---|---|
| Natriumlaurylethersulfat | 12% |
| (z.B. Texapon NSO, Fa. Cognis Deutschland GmbH) | |
| Cocamidopropylbetain | 2% |
| (z.B. Dehyton K, Fa. Cognis Deutschland GmbH) | |
| Natriumchlorid | 1,4% |
| Citronensäure | 1,3% |
| | |
| Phenoxyethanol, Methyl-, Ethyl-, Butyl-, | |
| und Propylparaben | 0,5% |
| Wasser | 82,8% |

Als Ergebnis resultierte eine modifizierte Shampoo-Grundmasse. Der pH-Wert dieser modifizierten Shampoo-Grundmasse lag bei etwa 6. Hieraus wurden 100 mL einer 20 Gew.%-igen wässrigen Shampoo-Lösung hergestellt. Mit dieser Shampoo-Lösung wurden 2 Haarsträhnchen jeweils für 2 Minuten gewaschen und anschließend 20 Sekunden unter fließendes, handwarmes Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.

Die Geruchsnoten wurden jeweils wie folgt beurteilt: sehr stark strahlend, moschusartig, exaltierend, weich holzig, erinnert an den Geruch von Moschuskörnern.

### Anwendungsbeispiel 4: Weichspüler

Die Parfümkomposition aus Anwendungsbeispiel 1 (modifizierte Parfümkomposition A) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% | 5,5% |
| (z.B. Rewoquat WE 18, Fa. Witco Surfactants GmbH) | |
| Alkyldimethylbenzylammoniumchlorid, ca. 50% | 0,2% |
| (z.B. Preventol R50, Fa. Bayer AG) | |
| Farblösung, ca. 1%-ig | 0,3% |
| | |
| Wasser | 94,0% |

Als Ergebnis resultierte eine modifizierte Weichspüler-Grundmasse. Der pH-Wert dieser modifizierten Weichspüler-Grundmasse lag im Bereich von 2 bis 3. Zwei Stofflappen wurden mit 370 g einer 1%-igen wässrigen (modifizierten) Weichspüler-Lösung in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweißt, der andere zum Trocknen aufgehängt. Anschließend wurden beide Lappen durch ein Panel geruchlich beurteilt.

Die Geruchsnoten wurden jeweils wie folgt beurteilt: blumig, holzig, frisch, strahlend, moschusartig und holzige Aspekte mit leichten süßen Untertönen, abgerundeter und harmonischer Geruchseindruck.

### Anwendungsbeispiel 5: Waschpulver

Die Parfümölkomposition aus Anwendungsbeispiel 2 (modifizierte Parfümkomposition B) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Silikonöl (auf Zeolith als Trägermaterial) | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis | |
| (methylen)]]tetrakis-phosphonsäure, Natriumsalz) | |
| Optischer Aufheller | 0,2 % |
| Na-Sulfat | 6,5 % |
| Protease | 0,4 % |
| Natriumperborattetrahydrat | 22,0 % |
| TAED | 1,0 % |

Als Ergebnis resultierte eine modifizierte Waschpulver-Grundmasse. Zwei Stofflappen wurden mit 370 g einer 1%-igen wässrigen (modifizierten) Waschpulverlauge (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt, der andere zum Trocknen aufgehängt. Anschließend wurden beide Lappen durch ein Panel geruchlich beurteilt.

Die Geruchsnoten wurden jeweils wie folgt beurteilt: blumig, strahlend, moschusartig und natürliche Note mit leichten süßen und holzigen Untertönen, abgerundeter und harmonischer Geruchseindruck

## Patentansprüche

1. Mischung umfassend oder bestehend aus zwei oder mehr Verbindungen ausgewählt aus der Formel (la) oder (Ib) 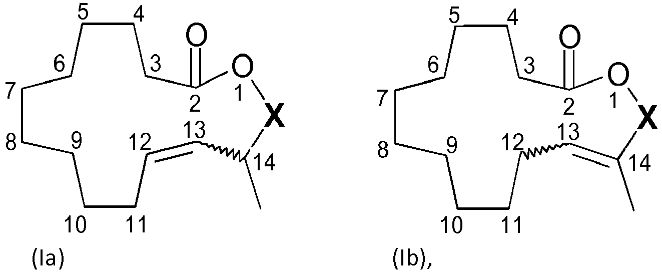 wobei unabhängig voneinander in jeder der Formeln (la) und (Ib) X eine Methylen- oder Ethylen-Gruppe bedeutet, und die gewellte Linie bedeutet, dass die Konfiguration an der zugeordneten Doppelbindung E oder Z ist und wobei in der Formel (la) die Konfiguration am Kohlenstoffatom mit der Nummer 14 R oder S ist.

2. Mischung nach Anspruch 1, wobei die Verbindungen (la) und (Ib) ausgewählt sind aus der Gruppe bestehend aus:
| **Formel** | **X** | **E** | **R/S** | **Strukturformel** |
|---|---|---|---|---|
| (Ia-1) | CH₂ | E | 14R | 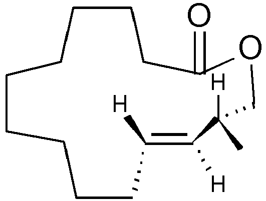 |
| (Ia-2) | CH₂ | E | 14S | 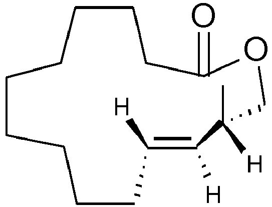 |
| (Ia-3) | C₂H₄ | E | 14S | 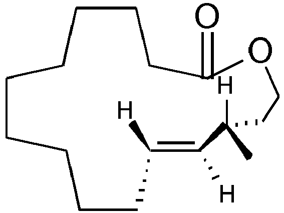 |
| (Ia-4) | C₂H₄ | E | 14R | 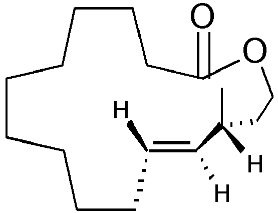 |
| (Ia-5) | CH₂ | Z | 14S | 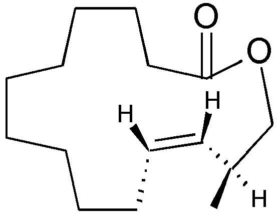 |
| (Ia-6) | CH₂ | Z | 14R | 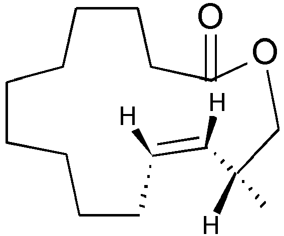 |
| (Ia-7) | C₂H₄ | Z | 14R | 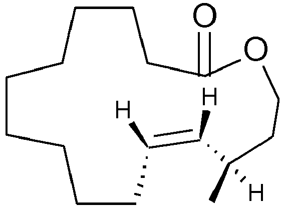 |
| (Ia-8) | C₂H₄ | Z | 14S | 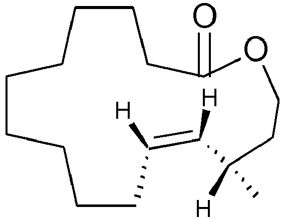 |
| (Ib-1) | CH₂ | Z | --- | 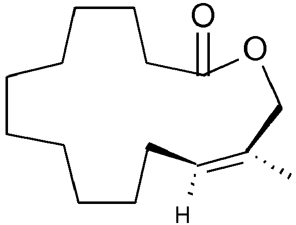 |
| (Ib-2) | CH₂ | Z | --- | 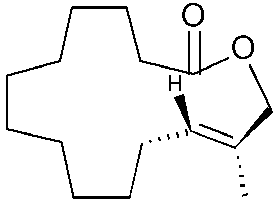 |
| (Ib-3) | CH₂ | E | --- | 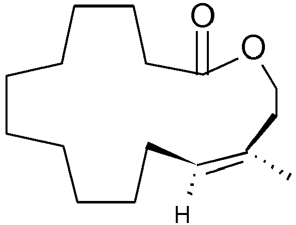 |
| (Ib-4) | C₂H₄ | Z | --- | 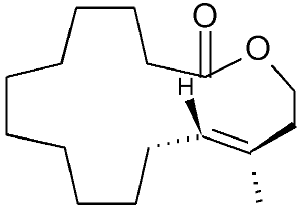 |

3. Mischung nach Anspruch 2,
- umfassend eine Verbindung oder mehr Verbindungen der Formel (la) und eine Verbindung oder mehr Verbindungen der Formel (Ib), wobei X für sämtliche der Verbindungen der Formel (la) und (Ib) die gleiche Bedeutung hat und eine Methylen- oder Ethylen-Gruppe bedeutet, und/oder
- umfassend zwei oder mehr Verbindungen der Formel (la), wobei X für beide oder sämtliche dieser Verbindungen die gleiche Bedeutung hat und eine Methylengruppe bedeutet, und/oder
- umfassend ein E-Isomer sowie ein Z-Isomer einer Verbindung der Formel (la), wobei X sowohl für das E-Isomer als auch für das Z-Isomer die gleiche Bedeutung hat und eine Methylen- oder Ethylen-Gruppe bedeutet, und/oder
- umfassend beide Enantiomere eines Enantiomerenpaares von Verbindungen der Formel (la) wie in einem der Ansprüche 1 oder 2 definiert, vorzugsweise ein Racemat zweier Enantiomere von Verbindungen der Formel (la) wie in einem der Ansprüche 1 oder 2 definiert, und/oder
- umfassend ein E-Isomer und ein Z-Isomer einer Verbindung der Formel (Ib), wobei X sowohl für das E-Isomer als auch für das Z-Isomer die gleiche Bedeutung hat und eine Methylen- oder Ethylen-Gruppe bedeutet.

4. Erzeugnis bestehend aus oder umfassend als Komponente (a)
- eine, zwei oder mehr Verbindungen gemäß einem der Ansprüche 1 oder 2, vorzugsweise zumindest eine, zwei oder mehr Verbindungen der Formel (la), wobei X für die eine, beide oder sämtliche dieser Verbindungen die gleiche Bedeutung hat und eine Methylengruppe bedeutet, und/oder
- ein E-Isomer sowie ein Z-Isomer einer Verbindung der Formel (la), wobei X sowohl für das E-Isomer als auch für das Z-Isomer die gleiche Bedeutung hat und eine Methylen- oder Ethylen-Gruppe bedeutet, und/oder
- beide Enantiomere eines Enantiomerenpaares von Verbindungen der Formel (la) wie in einem der Ansprüche 1 oder 2 definiert, vorzugsweise ein Racemat zweier Enantiomere von Verbindungen der Formel (la) wie in einem der Ansprüche 1 oder 2 definiert, und/oder
- ein E-Isomer und ein Z-Isomer einer Verbindung der Formel (Ib), wobei X sowohl für das E-Isomer als auch für das Z-Isomer die gleiche Bedeutung hat und eine Methylen- oder Ethylen-Gruppe bedeutet
sowie
ein, zwei oder mehr weitere Bestandteile.

5. Erzeugnis nach Anspruch 4, umfassend als Komponente (b) eine, zwei oder mehrere Verbindungen, die keine Verbindungen gemäß einem der Ansprüche 1 oder 2 sind, wobei diese Verbindung bzw. diese Verbindungen ausgewählt sind aus der Gruppe bestehend aus Riechstoffen, wobei
(I) die Gesamtmenge an Verbindungen gemäß einem der Ansprüche 1 oder 2 in dem Erzeugnis ausreicht, eine oder mehrere der Geruchsnoten moschusartig, strahlend, weich, holzig, erogen, blumig und exaltierend zu vermitteln, und/oder
(II) die Gesamtmenge an Verbindungen der Komponente (b) in dem Erzeugnis ausreicht, eine, mehrere oder sämtliche der Noten holzig, blumig, frisch, grün, fruchtig, moschusartig und würzig-balsamisch zu vermitteln, vorzugsweise eine oder sämtliche der Noten holzig und blumig, wobei vorzugsweise die Gesamtmenge an Verbindungen gemäß einem der Ansprüche 1 oder 2 in dem Erzeugnis ausreicht, eine oder mehrere dieser Noten zu verstärken und/oder zu modifizieren, und/oder
(III) die Gesamtmenge an Verbindungen gemäß einem der Ansprüche 1 oder 2 in dem Erzeugnis ausreicht, dem Erzeugnis einen Eindruck von Frische, Sauberkeit, Harmonie, Ausstrahlung und/oder Natürlichkeit zu verleihen, und/oder
(IV) die Gesamtmenge an Verbindungen gemäß einem der Ansprüche 1 oder 2 in dem Erzeugnis ausreicht, um im Vergleich zu einem Vergleichserzeugnis, das bei ansonsten identischer Zusammensetzung keine Verbindungen gemäß einem der Ansprüche 1 oder 2 umfasst, einen intensiveren, harmonischeren, hochwertigeren, helleren, weicheren, abgerundeteren und/oder natürlicheren Geruchseindruck zu bewirken.

6. Erzeugnis nach Anspruch 5, wobei
- die Gesamtmenge an Verbindungen gemäß einem der Ansprüche 1 oder 2 im Bereich von 0,00001 bis 99,9 Gew.-%, vorzugsweise 0,001 bis 70 Gew.-% und besonders bevorzugt 0,01 bis 50 Gew.-% liegt, bezogen auf die Gesamtmenge des Erzeugnisses, und/oder
- das Gewichtsverhältnis der Gesamtmenge an Verbindungen gemäß einem der Ansprüche 1 oder 2 zu der Gesamtmenge an sonstigen Riechstoffen, die keine Verbindungen sind, wie in einem der Ansprüche 1 bis 2 definiert, im Bereich von 1:1000 bis 1:0,5 liegt, bezogen auf die Gesamtmenge des Erzeugnisses.

7. Erzeugnis nach einem der Ansprüche 4 bis 6, umfassend als Komponente (b) eine, zwei oder mehrere Verbindungen, die keine Verbindungen gemäß einem der Ansprüche 1 oder 2 sind, wobei diese Verbindung bzw. diese Verbindungen ausgewählt sind aus der Gruppe bestehend aus Riechstoffen,
mit der Maßgabe, dass
- die Verbindung bzw. eine, zwei oder mehrere der Verbindungen eine Moschusriechstoffverbindung ist bzw. Moschusriechstoffverbindungen sind, vorzugsweise eine makrocyclische Moschusriechstoffverbindung ist bzw. makrocyclische Moschusriechstoffverbindungen sind, oder
- die Verbindung bzw. eine, zwei oder mehrere der Verbindungen ein Riechstoff bzw. Riechstoffe mit holziger Geruchsnote ist bzw. sind, oder
- die Verbindung bzw. eine, zwei oder mehrere der Verbindungen ein Riechstoff bzw. Riechstoffe mit blumiger Geruchsnote ist bzw. sind, oder
- die Verbindung bzw. eine, zwei oder mehrere der Verbindungen ein Riechstoff bzw. Riechstoffe mit grün, fruchtiger Geruchsnote ist bzw. sind.

8. Erzeugnis nach einem der Ansprüche 4 bis 7, umfassend (c) einen oder mehrere Trägerstoffe für die Komponente (a) und/oder die Komponente (b).

9. Erzeugnis nach Anspruch 8,, wobei der eine bzw. einer, mehr als einer oder sämtliche der mehreren Trägerstoffe der Komponente (c) ausgewählt ist bzw. sind aus der Gruppe bestehend aus (A) porösen anorganischen Materialien und (B) organischen Materialien, wobei das bzw. die organischen Materialien vorzugsweise Kunststoffe oder sonstige organische Polymere sind oder umfassen.

10. Erzeugnis nach einem der Ansprüche 4 bis 9,, umfassend (d) ein, zwei oder mehr Verbindungen ausgewählt aus der Gruppe bestehend aus Wasser, Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat und Triacetin.

11. Erzeugnis nach einem der Ansprüche 4 bis 10,, umfassend (e) ein, zwei oder mehr Bestandteile ausgewählt aus der Gruppe bestehend aus
(e1) Tensiden und sonstigen Grund-, Hilfs- und Zusatzstoffen zum Einsatz in parfümierten Erzeugnissen sowie
(e2) Grund-, Hilfs- und Zusatzstoffen zum Einsatz in Erzeugnissen für die Ernährung, die Mundpflege oder den Genuss.

12. Erzeugnis nach einem der Ansprüche 4 bis 11, ausgewählt aus der Gruppe bestehend aus Shampoos, Parfüm-Extraits, Eau de Parfums, Eau de Toilettes, Rasierwässern, Eau de Colognes, Pre-shave-Produkten, Splash-Colognes, parfümierten Erfrischungstüchern, sauren, alkalischen und neutralen Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachsen und Polituren, Körperpflegemitteln, Handcremes und -lotionen, Fußcremes und -lotionen, Enthaarungscremes und -lotionen, After-shave-Cremes und -lotionen, Bräunungscremes und - lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

13. Verbindung der Formel (la) 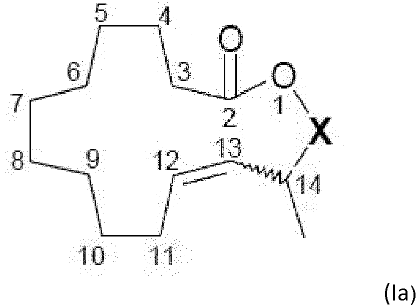 wobei X eine Methylen- oder Ethylen-Gruppe bedeutet, und die gewellte Linie bedeutet, dass die Konfiguration an der zugeordneten Doppelbindung E oder Z ist und wobei die Konfiguration am Kohlenstoffatom mit der Nummer 14 R oder S ist.

14. Verbindung ausgewählt aus der Gruppe bestehend aus
| **Formel** | **X** | **E** | **R/S** | **Strukturformel** |
|---|---|---|---|---|
| (Ia-1) | CH₂ | E | 14R | 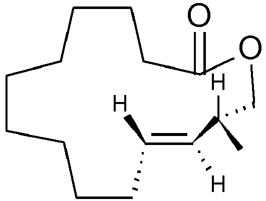 |
| (Ia-2) | CH₂ | E | 14S | 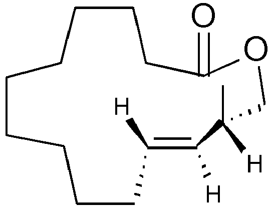 |
| (Ia-3) | C₂H₄ | E | 14S | 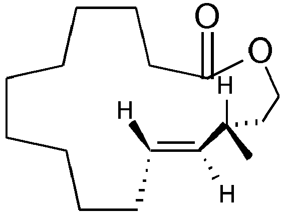 |
| (Ia-4) | C₂H₄ | E | 14R | 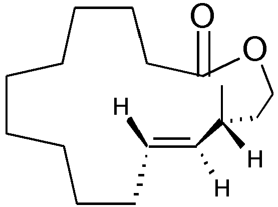 |
| (Ia-5) | CH₂ | Z | 14S | 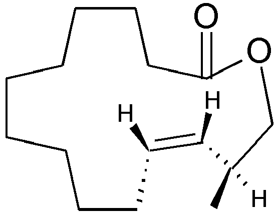 |
| (Ia-6) | CH₂ | Z | 14R | 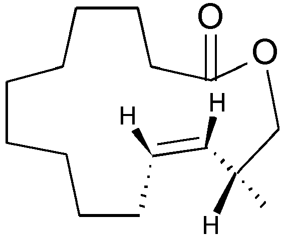 |
| (Ia-7) | C₂H₄ | Z | 14R | 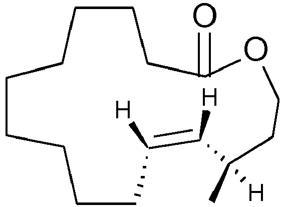 |
| (Ia-8) | C₂H₄ | Z | 14S | 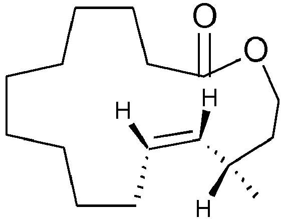 |

15. Verwendung einer Verbindung gemäß Ansprüche 13 oder 14 oder einer Mischung gemäß Ansprüche 1 bis 3 oder eines Erzeugnisses nach einem der Ansprüche 4 bis 12
- als Riechstoff bzw. als Riechstoffmischung bzw. als Riechstofferzeugnis, vorzugsweise mit einer oder mehreren Geruchsnoten ausgewählt aus der Gruppe bestehend aus moschusartig, strahlend, weich, holzig, erogen, blumig und exaltierend, bevorzugt mit der Geruchsnote moschusartig, und/oder
- zum Ersetzen eines Moschusriechstoffs, einer Moschusriechstoffmischung oder eines Moschusriechstofferzeugnisses mit geringerem Haftungsvermögen und/oder geringerer Substantivität und/oder geringerer Bioabbaubarkeit, und/oder
- zum Verstärken und/oder Modifizieren einer Geruchsnote, vorzugsweise einer Geruchsnote ausgewählt aus der Gruppe bestehend aus moschusartig, strahlend, weich, holzig, erogen, blumig und exaltierend, bevorzugt mit der Geruchsnote moschusartig, und/oder
- zum Versehen von Haut und/oder textilen Fasern mit einer Geruchsnote oder mehreren Geruchsnoten ausgewählt aus der Gruppe bestehend aus moschusartig, strahlend, weich, holzig, erogen, blumig und exaltierend, bevorzugt mit der Geruchsnote moschusartig, und/oder
- als Mittel zum Erhöhen der Substantivität und/oder Retention einer Riechstoffmischung und/oder als Fixateur und/oder als Mittel zum Erhöhen des über einer tensidhaltigen wässrigen Lösung wahrgenommenen Geruches anderer Riechstoffe.

16. Verfahren zur Herstellung einer Verbindung der Formel (la) oder (Ib) wie in einem der Ansprüche 1 und 2 definiert, umfassend die Schritte:
- Herstellen oder Bereitstellen einer Verbindung der Formel (IV), 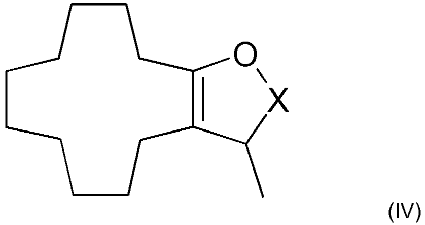 wobei X eine Methylen- oder Ethylen-Gruppe bedeutet
- Umsetzen der Verbindung der Formel (IV) in einem oder mehreren Schritten zur Verbindung der Formel (la) bzw. (Ib).

17. Verwendung einer Verbindung der Formel (IV) 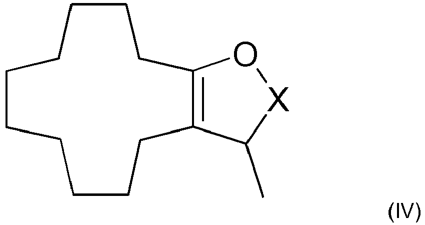 wobei X eine Methylen- oder Ethylen-Gruppe bedeutet, als Intermediat bei der Herstellung einer Verbindung der Formel (la) oder (Ib) wie in einem der Ansprüche 1 und 2 definiert.

## Claims

1. Mixture comprising or consisting of two or more compounds selected from formula (Ia) or (Ib) 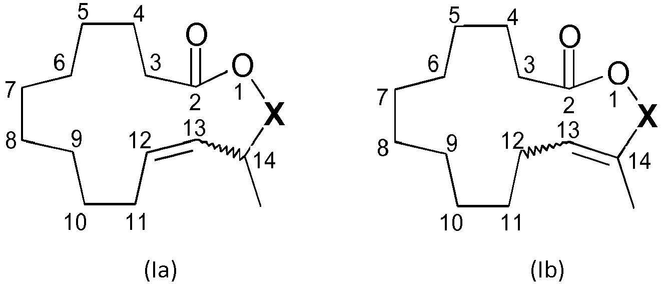 wherein independently in each of the formulae (Ia) and (Ib) X represents a methylene or ethylene group, and the wavy line indicates that the configuration on the associated double bond is E or Z, and in the formula (Ia) the configuration on the carbon atom numbered 14 is R or S.

2. A mixture according to claim 1, wherein the compounds (Ia) and (Ib) are selected from the group consisting of:
| **Formula** | **X** | **E** | **R/S** | **Structure formula** |
|---|---|---|---|---|
| (Ia-1) | CH₂ | E | 14R | 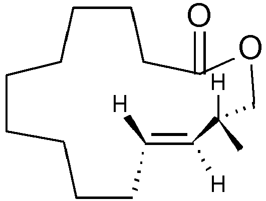 |
| (Ia-2) | CH₂ | E | 14S | 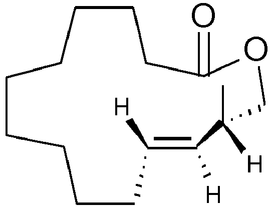 |
| (Ia-3) | C₂H₄ | E | 14S | 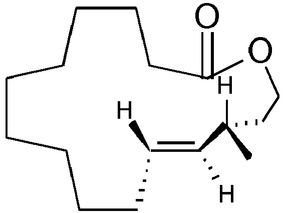 |
| (Ia-4) | C₂H₄ | E | 14R | 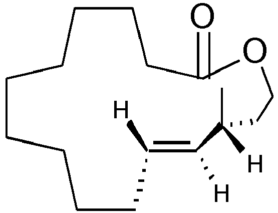 |
| (Ia-5) | CH₂ | Z | 14S | 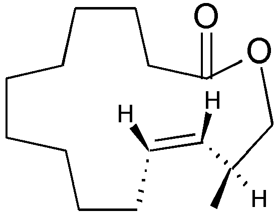 |
| (Ia-6) | CH₂ | Z | 14R | 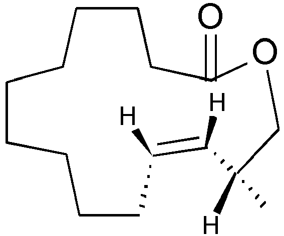 |
| (Ia-7) | C₂H₄ | Z | 14R | 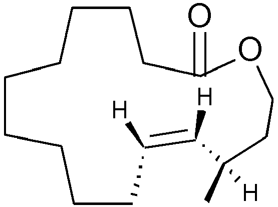 |
| (Ia-8) | C₂H₄ | Z | 14S | 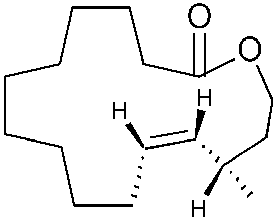 |
| (Ib-1) | CH₂ | Z | --- | 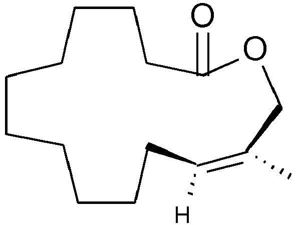 |
| (Ib-2) | CH₂ | Z | --- | 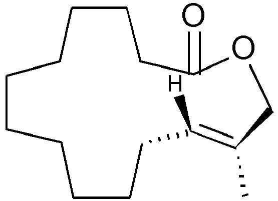 |
| (Ib-3) | CH₂ | E | --- | 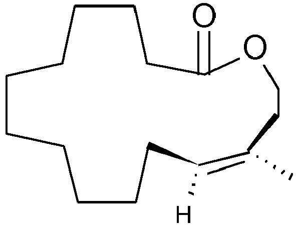 |
| (Ib-4) | C₂H₄ | Z | --- | 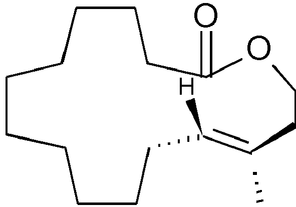 |

3. Mixture according to claim 2,
- comprising one or more compounds of formula (Ia) and one or more compounds of formula (Ib) wherein X has the same meaning for all of the compounds of formula (Ia) and (Ib) and represents a methylene or ethylene group, and/or
- comprising two or more compounds of formula (Ia), wherein X has the same meaning for both or all of these compounds and represents a methylene group, and/or
- comprising an E isomer and a Z isomer of a compound of formula (Ia) wherein-X has the same meaning for both the E isomer and the Z isomer and represents a methylene or ethylene group, and/or
- comprising both enantiomers of an enantiomeric pair of compounds of formula (Ia) as defined in any one of Claims 1 or 2, preferably a racemate of two enantiomers of compounds of formula (Ia) as defined in any one of Claims 1 or 2, and/or
- comprising an E isomer and a Z isomer of a compound of formula (Ib) wherein X is the same for both the E isomer and the Z isomer and represents a methylene or ethylene group.

4. Product consisting of or comprising as component (a)
- one, two or more compounds according to one of claims 1 or 2, preferably at least one, two or more compounds of formula (Ia) wherein X has the same meaning for one, both or all of these compounds and is a methylene group, and/or
- an E isomer and a Z isomer of a compound of formula (Ia) wherein X has the same meaning for both the E isomer and the Z isomer and represents a methylene or ethylene group, and/or
- both enantiomers of a pair of enantiomers of compounds of formula (Ia) as defined in one of Claims 1 or 2, preferably a racemate of two enantiomers of compounds of formula (Ia) as defined in one of Claims 1 or 2, and/or
- an E isomer and a Z isomer of a compound of formula (Ib) wherein X is the same for both the E isomer and the Z isomer and represents a methylene or ethylene group
as well as
one, two or more other ingredients.

5. A product according to claim 4 comprising as component (b)
one, two or more compounds which are not compounds according to any of Claims 1 or 2, said compound or compounds being selected from the group consisting of perfumes, wherein
(I) the total quantity of compounds in the product, according to any one of claims 1 or 2, is sufficient to convey one or more of the olfactory notes musky, radiant, soft, woody, erogenous, floral and exalting; and/or
(II) the total amount of compounds of component (b) in the product is sufficient to impart one, more or all of the notes woody, floral, fresh, green, fruity, musky and spicy-balsamic, preferably one or all of the notes woody and floral, preferably the total amount of compounds according to any one of claims 1 or 2 in the product is sufficient to reinforce and/or modify one or more of those notes, and/or
(III) the total quantity of compounds in any one of Claims 1 or 2 in the product is sufficient to give the product an impression of freshness, cleanliness, durability, radiance and/or naturalness; and/or
(IV) the total quantity of compounds according to any of Claims 1 or 2 in the product is sufficient to give a more intense, harmonious, superior, lighter, softer, rounded and/or natural odour impression compared to a reference product which, if otherwise identical in composition, does not contain compounds according to any of Claims 1 or 2.

6. Product according to claim 5, where
- the total amount of compounds according to any one of Claims 1 or 2 is in the range of from 0.00001 to 99.9% by weight, preferably from 0.001 to 70% by weight, and in particular preferably from 0.01 to 50% by weight, based on the total amount of the product, and/or
- the weight ratio of the total amount of compounds according to any one of Claims 1 or 2 to the total amount of other perfumes which are not compounds as defined in any one of Claims 1 to 2 ranges from 1:1000 to 1:0.5 based on the total amount of the product.

7. A product according to any one of Claims 4 to 6 comprising as component (b) one, two or more compounds other than compounds according to any one of Claims 1 or 2, said compound or compounds being selected from the group consisting of fragrances, provided that
- the compound or one, two or more of the compounds is or are a musk fragrance compound, preferably a macrocyclic musk fragrance compound or are macrocyclic musk fragrance compounds, or
- the compound or one, two or more of the compounds is or are a perfuming substance or substances with a woody odour, or
- the compound or one, two or more of the compounds is or are a perfuming substance or substances with a floral odour, or
- the compound or one, two or more of the compounds is or are a perfuming substance or substances with a green, fruity odour.

8. A product according to any of Claims 4 to 7 comprising (c) one or more carriers for component (a) and/or component (b).

9. A product according to claim 8, wherein the one or more, more than one, or all of the carriers of component (c) is selected from the group consisting of (A) porous inorganic materials and (B) organic materials, wherein the organic material or materials are preferably plastics or other organic polymer.

10. A product according to any of Claims 4 to 9 comprising (d) one, two or more compounds selected from the group consisting of water, ethanol, isopropanol, diethylene glycol monoethyl ether, glycerin, propylene glycol, 1,2-butylene glycol, dipropylene glycol, diethyl phthalate, triethyl citrate, isopropyl myristate and triacetin.

11. A product according to any of Claims 4 to 10 comprising (e) one, two or more ingredients selected from the group consisting of
(e1) surfactants and other basic and auxiliary substances and additives for use in perfumed products, and
(e2) raw materials, consumables and additives for use in products intended for human consumption, oral hygiene or human consumption.

12. A product according to any of Claims 4 to 11 selected from the group consisting of shampoos, perfume extracts, eau de parfums, eau de toilettes, shaving lotions, eau de colognes, pre-shave products, splash colognes, perfumed refreshing wipes, acidic, alkaline and neutral detergents, textile fresheners, ironing aids, liquid detergents, powdery detergents, laundry pretreatment agents, fabric softeners, washing soaps, washing tablets, Disinfectants, surface disinfectants, air fresheners, aerosol sprays, waxes and polishes, body care products, hand creams and lotions, foot creams and lotions, depilatory creams and lotions, After-shave creams and lotions, tanning creams and lotions, hair care products, deodorants and antiperspirants, decorative cosmetic products, candles, lamp oils, incense sticks, insecticides, repellents and fuels.

13. A compound of formula (Ia) 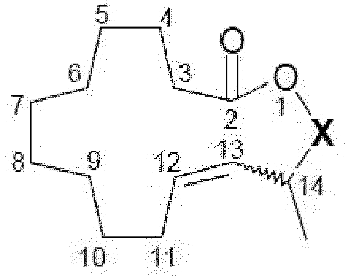 wherein X represents a methylene or ethylene group, and the wavy line indicates that the configuration on the associated double bond is E or Z and wherein the configuration on the carbon atom number 14 is R or S.

14. A compound selected from the group consisting of:
| **Formula** | **X** | **E** | **R/S** | **Structure formula** |
|---|---|---|---|---|
| (Ia-1) | CH₂ | E | 14R | 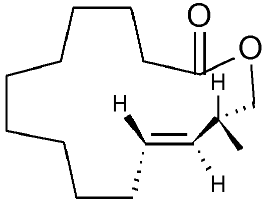 |
| (Ia-2) | CH₂ | E | 14S | 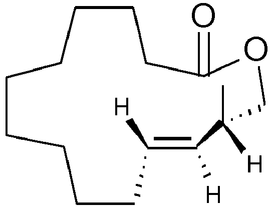 |
| (Ia-3) | C₂H₄ | E | 14S | 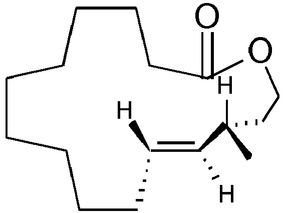 |
| (Ia-4) | C₂H₄ | E | 14R | 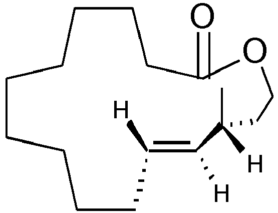 |
| (Ia-5) | CH₂ | Z | 14S | 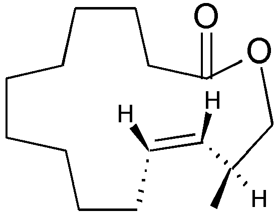 |
| (Ia-6) | CH₂ | Z | 14R | 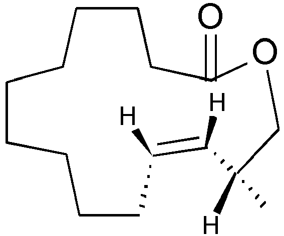 |
| (Ia-7) | C₂H₄ | Z | 14R | 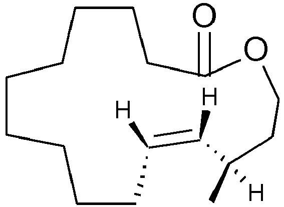 |
| (Ia-8) | C₂H₄ | Z | 14S | 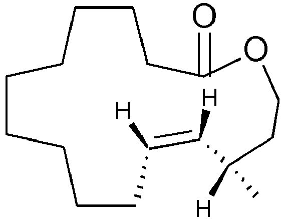 |

15. Use of a compound according to claims 13 or 14 or a mixture according to claims 1 to 3 or a product according to any of claims 4 to 12
- as an odorant or as an odorant mixture or as an odorant product, preferably with one or more odor notes selected from the group consisting of musky, radiant, soft, woody, erogenous, floral and exalting, preferably with the odor note musky, and/or
- for replacing a musk odorant, a musk odorant mixture or a musk odorant product with lower adhesion and/or lower substantivity and/or lower biodegradability, and/or
- for reinforcing and/or modifying an olfactory note, preferably a fruity note selected from the group consisting of musky, radiant, soft, woody, erogenous, floral and exalted, preferably with the olfactory note mossy, and/or
- for providing skin and/or textile fibers with an odour note or several odour notes selected from the group consisting of musky, radiant, soft, woody, erogenous, floral and exalting, preferably with the fruity note musky, and/or
- as an agent for increasing the substantivity and/or retention of an odorant mixture and/or as a fixer and/or as an agent for increasing the odor of other odorants perceived over a ten-side-containing aqueous solution.

16. A process for preparing a compound of formula (Ia) or (Ib) as defined in any of Claims 1 and 2 comprising the steps:
- preparing or providing a compound of formula (IV), 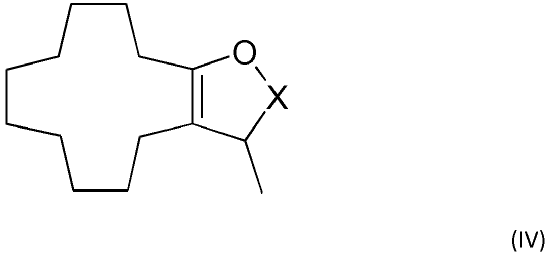 wherein X represents a methylene or ethylene group
- reacting the compound of formula (IV) in one or more steps to give the compound of formula (Ia) or (lb), respectively.

17. Use of a compound of formula (IV) 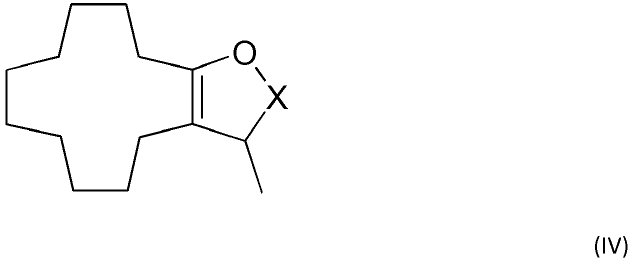 wherein X represents a methylene or ethylene group, as an intermediate in the preparation of a compound of formula (Ia) or (Ib) as defined in any of Claims 1 and 2.

## Revendications

1. Un mélange comprenant ou consistant en deux ou plusieurs composés choisis parmi les formules (la) ou (Ib) 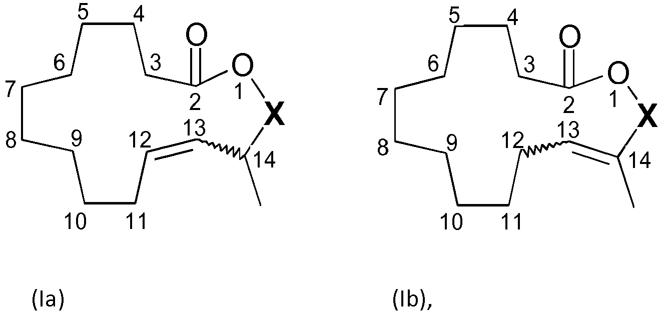 où indépendamment dans chacune des formules (la) et (Ib) X est un groupe méthylène ou éthylène, et la ligne ondulée signifie que la configuration à la double liaison associée est E ou Z et formule (la) dans laquelle la configuration à l'atome de carbone numéro 14 est R ou S.

2. Mélange selon la revendication 1, dans lequel les composés (la) et (Ib) sont choisis dans le groupe constitué par
| **Formule** | **X** | **E** | **R/S** | **Formule structurale** |
|---|---|---|---|---|
| (Ia-1) | CH₂ | E | 14R | 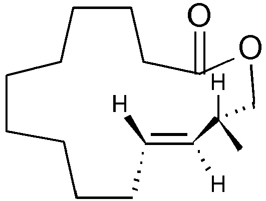 |
| (Ia-2) | CH₂ | E | 14S | 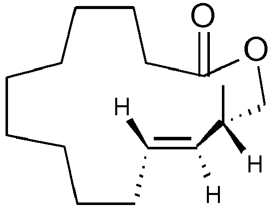 |
| (Ia-3) | C₂H₄ | E | 14S | 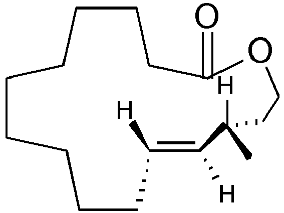 |
| (Ia-4) | C₂H₄ | E | 14R | 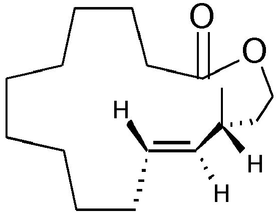 |
| (Ia-5) | CH₂ | Z | 14S | 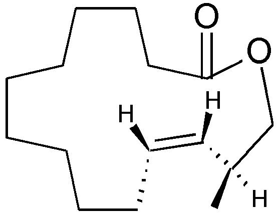 |
| (Ia-6) | CH₂ | Z | 14R | 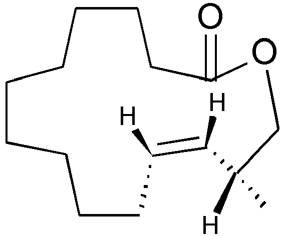 |
| (Ia-7) | C₂H₄ | Z | 14R | 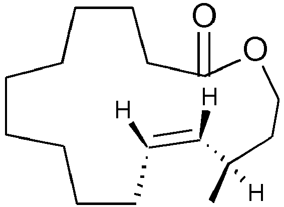 |
| (Ia-8) | C₂H₄ | Z | 14S | 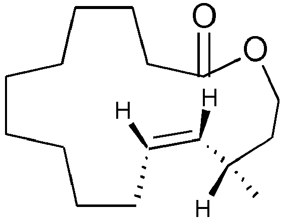 |
| (Ib-1) | CH₂ | Z | - | 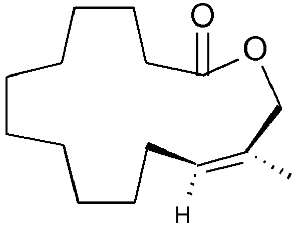 |
| (Ib-2) | CH₂ | Z | --- | 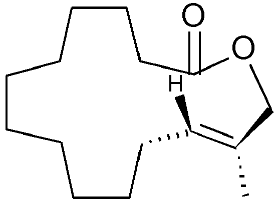 |
| (Ib-3) | CH₂ | E | --- | 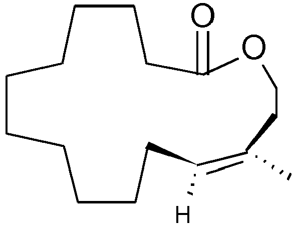 |
| (Ib-4) | C₂H₄ | Z | --- | 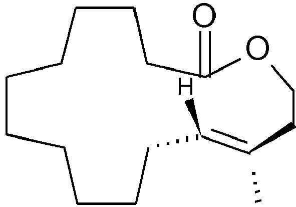 |

3. Mélange selon la revendication 2,
- comprenant un ou plusieurs composés de formule (la) et un ou plusieurs composés de formule (Ib) dans laquelle X est identique pour tous les composés de formule (la) et (Ib) et représente un groupe méthylène ou éthylène ; et/ou
- comprenant deux ou plusieurs composés de formule (la) dans laquelle X a la même signification pour ces deux ou tous ces composés et représente un groupe méthylène; et/ou
- comprenant un isomère E et un isomère Z d'un composé de formule (la) dans laquelle X est identique pour l'isomère E et l'isomère Z et représente un groupe méthylène ou éthylène; et/ou
- comprenant les deux énantiomères d'une paire énantiomère de composés de formule (la) tels que définis dans l'une quelconque des revendications 1 ou 2, de préférence un racémate de deux énantiomères de composés de formule (la) tels que définis dans une quelconque des revendications 1 ou 2 ; et/ou
- comprenant un isomère E et un isomère Z d'un composé de formule (Ib) dans laquelle X est identique pour l'isomère E et l'isomère Z et représente un groupe méthylène ou éthylène.

4. Un produit consistant en ou comprenant en tant que composant (a)
- un, deux ou plusieurs composés selon l'une quelconque des revendications 1 ou 2, de préférence au moins un, deux ou plusieurs composés de formule (la) dans laquelle X a la même signification pour un, les deux ou tous ces composés et représente un groupe méthylène; et/ou
- un isomère E et un isomère Z d'un composé de formule (la) dans laquelle X est identique pour l'isomère E et l'isomère Z et représente un groupe méthylène ou éthylène; et/ou
- les deux énantiomères d'une paire d'énantiomères de composés de formule (la) tels que définis dans l'une des revendications 1 ou 2, de préférence un racémate de deux énantiomères de composés de formule (la) tels que définis dans une des revendications 1 ou 2; et/ou
- un isomère E et un isomère Z d'un composé de formule (Ib) dans laquelle X est identique pour l'isomère E et l'isomère Z et représente un groupe méthylène ou éthylène;
aussi bien que un, deux ou plusieurs autres ingrédients.

5. Produit selon la revendication 4 comprenant en tant que composant (b) un, deux ou plusieurs composés qui ne sont pas des composés selon l'une quelconque des revendications 1 ou 2, ledit composé ou lesdits composés étant choisis dans le groupe constitué des parfums; là où
(I) la quantité totale de composés dans le produit, telle que définie dans l'une des revendications 1 ou 2, est suffisante pour véhiculer une ou plusieurs des notes olfactives musquée, radieuse, douce, boisée, érogène, florale et exaltée ; et/ou
(II) la quantité totale de composés du composant (b) dans le produit est suffisante pour véhiculer une, plusieurs ou toutes les notes boisées, florales, fraîches, vertes, fruitées, musquées et épicées-balsamiques, de préférence une ou plusieurs des notes boisées et florales,
de préférence, la quantité totale de composés selon l'une quelconque des revendications 1 ou 2 dans le produit étant suffisante pour renforcer et/ou modifier une ou plusieurs de ces notes ; et/ou
(III) la quantité totale de composés dans l'une quelconque des revendications 1 ou 2 du produit est suffisante pour donner au produit une impression de fraîcheur, de propreté, d'harmonie, d'éclat et/ou de naturel ; et/ou
(IV) la quantité totale de composés selon l'une quelconque des revendications 1 ou 2 dans le produit est suffisante pour donner une impression d'odeur plus intense, harmonieuse, supérieure, plus légère, plus douce, arrondie et/ou naturelle par rapport à un produit de référence qui, s'il est autrement identique dans sa composition, ne contient aucun composé selon l'une des revendications 1 ou 2.

6. Produit selon la revendication 5, où la quantité totale de composés selon l'une quelconque des revendications 1 ou 2 est comprise entre 0,00001 et 99,9 % en poids, de préférence entre 0,001 et 70 % en poids, et en particulier entre 0,01 et 50 % en poids, par rapport à la quantité totale du produit ; et/ou
le rapport pondéral de la quantité totale de composés selon l'une quelconque des revendications 1 ou 2 à la quantité totale d'autres parfums qui ne sont pas des composés tels que définis dans l'une quelconque des revendications 1 à 2 est compris entre 1:1000 et 1:0,5 par rapport à la quantité totale du produit.

7. Un article selon l'une quelconque des revendications 4 à 6 comprenant comme composant (b) un, deux ou plusieurs composés qui ne sont pas des composés selon l'une quelconque des revendications 1 ou 2, ledit composé ou lesdits composés étant choisis dans le groupe constitué des parfums,
à condition que
- le composé ou un, deux ou plusieurs des composés est ou sont un composé de parfum de musc, de préférence un composé de parfum de musc macrocyclique ou sont des composés de parfum de musc macrocyclique ou
- le composé ou un, deux ou plusieurs des composés est ou sont un odorant ou des odorants avec une note d'odeur boisée ou
- le composé ou un, deux ou plusieurs des composés est ou sont un odorant ou des odorants avec une note d'odeur florale ou
- le composé ou un, deux ou plusieurs des composés est ou sont une substance parfumante ou des substances ayant une odeur verte et fruitée.

8. Un produit selon l'une quelconque des revendications 4 à 7 comprenant c) un ou plusieurs supports pour le composant a) et/ou le composant b).

9. Produit selon la revendication 8, dans lequel l'un ou plusieurs, plus d'un ou tous les supports du composant (c) sont choisis dans le groupe constitué par (A) des matériaux inorganiques poreux et (B) des matériaux organiques, le ou les matériaux organiques étant de préférence des matières plastiques ou autres polymères organiques.

10. Produit selon l'une quelconque des revendications 4 à 9 comprenant (d) un, deux ou plusieurs composés choisis dans le groupe constitué par l'eau, l'éthanol, l'isopropanol, l'éther monoéthylique de diéthylène glycol, le glycérol, le propylène glycol, le 1,2-butylène glycol, le dipropylène glycol, le phtalate de diéthyle, le citrate de triéthyle, le myristate d'isopropyle et la triacétine.

11. Un produit selon l'une quelconque des revendications 4 à 10 comprenant e) un, deux ou plusieurs membres choisis dans le groupe constitué par
e1) tensio-actifs et autres matières premières, auxiliaires et additifs utilisés dans les produits parfumés aussi bien que
e2) matières premières, consommables et additifs destinés à être utilisés dans les produits destinés à la consommation humaine, à l'hygiène buccale ou à la consommation humaine.

12. Produit selon l'une quelconque des revendications 4 à 11 choisi dans le groupe constitué par les shampooings, les extraits de parfum, les eaux de parfums, les eaux de toilette, les lotions à raser, les eaux de colognes, les produits de pré-rasage et les colognes à éclabousser, lingettes rafraîchissantes parfumées, détergents acides, alcalins et neutres, désodorisants textiles, produits de repassage, détergents liquides, détergents en poudre, détergents en poudre, agents de prétraitement du linge, assouplissants pour textiles, savons de lavage, comprimés de lavage, désinfectants, désinfectants de surface, désodorisants, aérosols en aérosol, cires et encaustiques, produits de soins corporels, crèmes et lotions pour les mains, crèmes et lotions pour les pieds, crèmes et lotions dépilatoires, crèmes et lotions après-rasage, crèmes et lotions de bronzage, produits de soins capillaires, déodorants et antisudorifiques, produits cosmétiques décoratifs, bougies, huiles pour lampes, bâtons d'encens, insecticides, répulsifs et carburants.

13. Composé de formule (la) 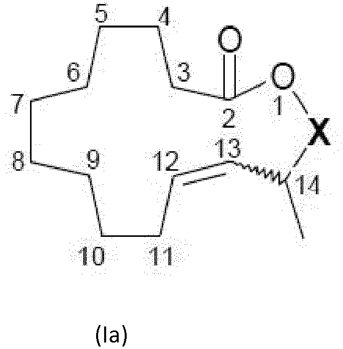 là où X est un groupe méthylène ou éthylène, et la ligne ondulée signifie que la configuration à la double liaison associée est E ou Z et dans laquelle la configuration à l'atome de carbone numéro 13 est R ou S.

14. Composé choisi dans le groupe constitué par
| **Formule** | **X** | **E** | **R/S** | **Formule structurale** |
|---|---|---|---|---|
| (Ia-1) | CH₂ | E | 14R | 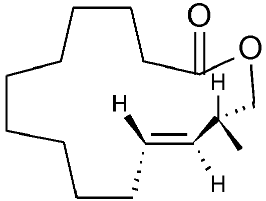 |
| (Ia-2) | CH₂ | E | 14S | 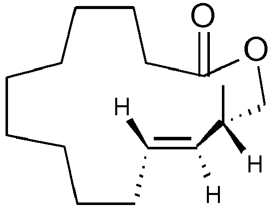 |
| (Ia-3) | C₂H₄ | E | 14S | 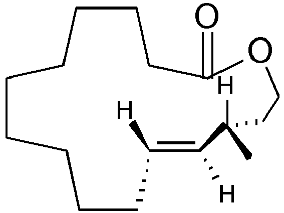 |
| (Ia-4) | C₂H₄ | E | 14R | 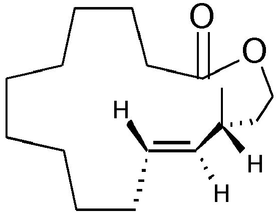 |
| (Ia-5) | CH₂ | Z | 14S | 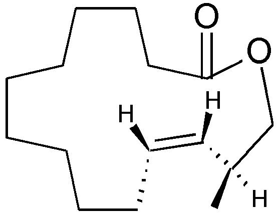 |
| (Ia-6) | CH₂ | Z | 14R | 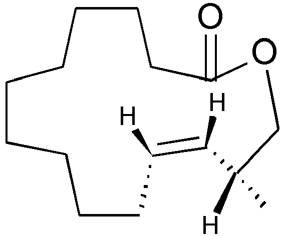 |
| (Ia-7) | C₂H₄ | Z | 14R | 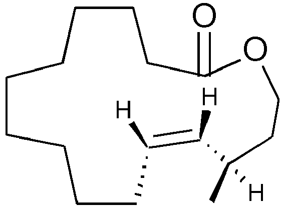 |
| (Ia-8) | C₂H₄ | Z | 14S | 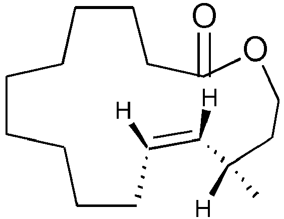 |

15. Utilisation d'un composé selon les revendications 13 ou 14 ou d'un mélange selon les revendications 1 à 3 ou d'un produit selon l'une quelconque des revendications 4 à 12 en tant que substance parfumante ou mélange de substances parfumantes ou en tant que produit de substance parfumante, de préférence avec une ou plusieurs notes olfactives choisies dans le groupe constitué par les notes musquées, radiantes, douces, boisées, boisées, érogènes, florales et exaltantes, de préférence avec la note olfactive musquée ; et/ou
- pour remplacer une odeur de musc, un mélange d'odeurs de musc ou un produit d'odeur de musc par une adhésion et/ou une substantivité et/ou une biodégradabilité moindre ; et/ou
- pour renforcer et/ou modifier une note olfactive, de préférence une note olfactive choisie dans le groupe constitué par les notes musquée, radieuse, douce, boisée, érogène, florale et exaltante, de préférence avec la note olfactive musquée ; et/ou
- pour conférer aux fibres cutanées et/ou textiles une ou plusieurs notes olfactives choisies dans le groupe constitué par les notes musquées, radieuses, douces, douces, ligneuses, érogènes, florales et exaltantes, de préférence avec la note olfactive musquée ; et/ou
- comme agent pour augmenter la substantivité et/ou la rétention d'un mélange d'odorants et/ou comme fixateur et/ou comme agent pour augmenter l'odeur d'autres odorants perçus par-dessus une solution aqueuse contenant un agent tensioactif.

16. Procédé de préparation d'un composé de formule (la) ou (Ib) tel que défini dans l'une quelconque des revendications 1 et 2 comprenant les étapes :
- préparation ou fourniture d'un composé de formule (IV), 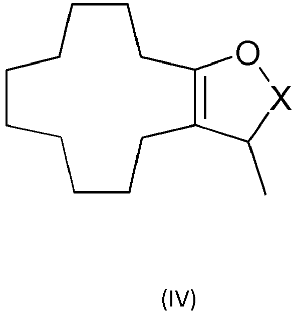 dans laquelle X représente un groupe méthylène ou éthylène
- la réaction du composé de formule (IV) en une ou plusieurs étapes pour donner le composé de formule (la) ou (Ib), respectivement.

17. Utilisation d'un composé de formule (IV) 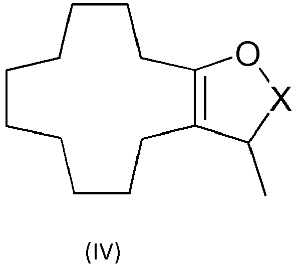 dans laquelle X représente un groupe méthylène ou éthylène comme intermédiaire dans la préparation d'un composé de formule (la) ou (Ib) tel que défini dans l'une quelconque des revendications 1 et 2.
